(19) Europäisches Patentamt
European Patent Office
Office européen des brevets

(11) Numéro de publication : **0 389 352 B1**

(12) # FASCICULE DE BREVET EUROPEEN

(45) Date de publication du fascicule du brevet : **13.04.94 Bulletin 94/15**

(21) Numéro de dépôt : **90400743.2**

(22) Date de dépôt : **20.03.90**

(51) Int. Cl.$^5$ : **C07D 211/32,** C07D 401/06, C07D 401/12, C07D 401/14, C07D 403/06, C07D 405/12, C07D 487/04, C07D 513/04

(54) **Dérivés fluoro-4 benzoiques, leurs procédés de préparation et les compositions pharmaceutiques qui les contiennent.**

Le dossier contient des informations techniques présentées postérieurement au dépôt de la demande et ne figurant pas dans le présent fascicule.

(30) Priorité : **21.03.89 FR 8903653**

(43) Date de publication de la demande : **26.09.90 Bulletin 90/39**

(45) Mention de la délivrance du brevet : **13.04.94 Bulletin 94/15**

(84) Etats contractants désignés : **AT BE CH DE DK ES FR GB GR IT LI LU NL SE**

(56) Documents cités : **EP-A- 0 013 612**

(56) Documents cités :
**EP-A- 0 070 053**
**EP-A- 0 184 258**
**CHEMICAL ABSTRACTS, vol. 112, no. 5, 29 janvier 1990, page 584, résumé no.35877z, Columbus, Ohio, US**

(73) Titulaire : **ADIR ET COMPAGNIE**
**1 rue Carle Hébert**
**F-92415 Courbevoie Cédex (FR)**

(72) Inventeur : **Lavielle, Gilbert**
**1 avenue Lilly**
**F-78170 La Celle Saint Cloud (FR)**
Inventeur : **Colpaert, Francis**
**36bis Bld. Carnot**
**F-78110 Le Vesinet (FR)**
Inventeur : **Laubie, Michel**
**35 avenue Foch**
**F-92420 Vaucresson (FR)**

**EP 0 389 352 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet des nouveaux dérivés fluoro-4 benzoïques, leurs procédés de préparation et les compositions pharmaceutiques qui les contiennent.

De nombreux composés fluoro-4 benzoïques, dérivés de la pipéridine ou de la pyrrolidine, possèdent des propriétés pharmacologiques intéressantes et sont décrits dans la littérature. En effet, des (fluoro-4 benzoyl)-4 alkyl-1 pipéridines qui sont des dérivés de la benzimidazolone, de la quinazoline, de la pyrido [1,2-a] pyrimidinone, de la thiadiazolo [3,2-a] pyrimidinone et de la pyrimido [2,1-b] [1,3] thiazinone sont connus comme étant des antagonistes de la sérotonine (Brevet US 4254127, Demandes de Brevet EP 013 612, EP 037 265, EP 070 053, EP 184 258). Ces composés trouvent leurs applications en thérapeutique comme agents antivasospastiques et antiagrégants ou comme psychotropes. Des dérivés de la [(fluoro-4 benzoyl)-4 pipéridino] alkyl théophylline, ayant des propriétés antisérotoniques, antihistaminiques, et béta-stimulantes, sont décrits dans la littérature. (Demande de Brevet EP 071 738). Des (fluoro-4 benzoyl)-4 pipéridino alkyl indoles possédant des propriétés antihypertensives, analgésiques et tranquillisantes (Brevet US 4 110 459 et demandes de Brevet EP 045 024 et EP 046 179) et des dérivés de la polyalcoxyphényl pyrrolidone substitués par un groupement (fluoro-4 benzoyl)-4 pipéridine et ayant des propriétés vasodilatatrices et hypotensives, sont aussi connus (Demande de Brevet EP 008 645). Certaines imides, dérivés de la (fluoro-4 benzoyl)-4 pipéridine ou des dérivés du [(fluoro-4 benzoyl)-4 pipéridino]-2 benzodioxanne sont des agents neuroleptiques (Demandes de Brevet EP 261 688 et Brevet US 4 129 655). D'autres dérivés de la [(fluoro-4 benzoyl)-4 pipéridino] pipéridine, utilisables pour le traitement de la démence et des séquelles des maladies cérébrovasculaires, sont décrits dans la demande de Brevet EP 229 391.

Des dérivés de l'hydroxypropoxy thiazole ou de l'hydroxypropoxy phényle ainsi que des dérivés de la pyrimidine substitués par un groupement fluoro-4 benzoïque sont doués des propriétés antagonistes al-adrénergiques et/ou antihypertensives. (Brevets US 4 616 017; US 4 539 318 et Demande de Brevet DE 3601731). Quelques dérivés de la fluoro-4 phénacyl pyrrolidine sont aussi décrits comme étant actifs au niveau du système nerveux central. (Chem.Pharm.Bull.,(1977),25(8),p.1911). Enfin des dérivés de (pipéridinylalkyl)-1-oxophtalazine ont été décrits en tant qu'agents cardiovasculaires ou antitumoraux (Chem. Abstr., vol. 112, n° 35877z, 1989).

Les composés de la présente invention se distinguent des autres dérivés fluoro-4 benzoïques décrits dans la littérature par leurs structures originales et par leurs propriétés pharmacologiques nouvelles.

Les composés de l'invention allient de puissantes propriétés 5-$HT_2$ antagonistes à des propriétés $\alpha_1$-antagonistes qui les rendent particulièrement utiles dans le traitement de l'hypertension ou des affections secondaires à l'hypertension, tout en assurant la protection de la paroi vasculaire. De plus, les composés de l'invention sont capables d'antagoniser spécifiquement des symptômes complexes induits chez l'animal par l'injection du 5-hydroxy tryptophane, ce qui laisse présager que ces nouveaux composés sont également antagonistes de la sérotonine au niveau des récepteurs du type 5-$HT_1$. Ils peuvent donc être utiles dans le traitement de l'anxiété. Les composés de l'invention possèdent aussi des propriétés antihistaminiques et trouvent également leur application comme agents antiallergiques.

La présente invention a plus particulièrement pour objet les dérivés fluoro-4 benzoïques de formule I:

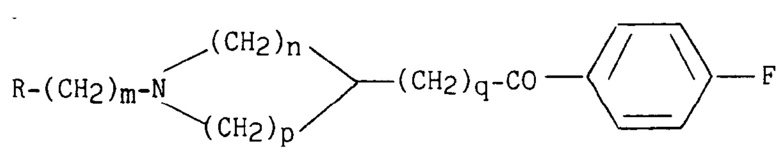

(I)

dans laquelle :

- m représente un nombre entier de 2 à 4,
- n et p identiques ou différents représentent chacun un nombre entier de 1 à 3, n et p ne représentant toutefois pas simultanément le nombre 3,
- q représente 0 ou 1

R représente :

. ou bien un groupement de formule (A):

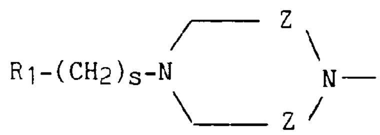

**(A)**

dans laquelle s représente un nombre entier de O à 4, Z représente un radical méthylène ou un radical carbonyle et $R_1$ représente soit un radical phényle (éventuellement substitué par un ou plusieurs atomes d'halogène, par un radical alcoyle inférieur linéaire ou ramifié contenant de 1 à 5 atomes de carbone ou un radical alcoxy inférieur contenant de 1 à 5 atomes de carbone) soit un radical diphénylméthylène (éventuellement substitué par un ou plusieurs atomes d'halogène, par un radical alcoyle inférieur ou un radical alcoxy inférieur), soit un cycle insaturé de cinq ou six chaînons comportant un ou deux atomes d'azote,

. ou bien un radical de formule (B)

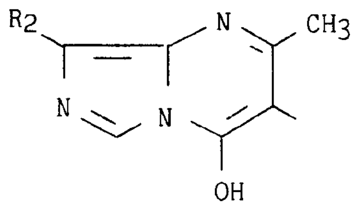

**(B)**

dans laquelle $R_2$ représente un radical carbamoyle, un radical cyano, un radical carboxy ou un radical alcoxycarbonyle contenant de 2 à 7 atomes de carbone,

. ou bien un radical dioxo-2,4 tétrahydro-1,2,3,4 quinazolinyle à la condition toutefois que, dans ce cas, n et p ne représentent pas simultanément le nombre 2,

. ou bien un radical oxo-1 phtalazinyle, à la condition que n et p ne représentent pas simultanément le nombre 2,

. ou bien un radical oxo-5 thiazolo [3,2-a] pyrimidinyle (éventuellement substitué par un ou plusieurs atomes d'halogène, par un radical alcoyle linéaire ou ramifié contenant de 1 à 5 atomes de carbone ou un radical alcoxy inférieur, contenant de 1 à 5 atomes de carbone), à la condition que n et p ne représentent pas simultanément le nombre 2,

. ou bien un groupement benzhydryloxy (dont les radicaux phényles sont éventuellement substitués par un ou plusieurs atomes d'halogène, par un radical alcoyle linéaire ou ramifié contenant de 1 à 5 atomes de carbone ou un radical alcoxy contenant de 1 à 5 atomes de carbone),

. ou bien un groupement de formule C :

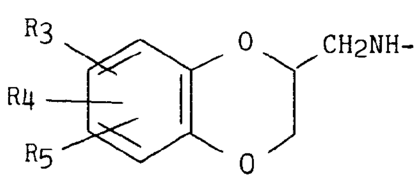

**(C)**

dans lequel $R_3$, $R_4$, $R_5$ identiques ou différents représentent chacun un atome d'halogène, un radical alcoxy de 1 à 5 atomes de carbone ou un radical alcoyle linéaire ou ramifié de 1 à 5 atomes de carbone,

leurs stéréoisomères possibles, et leurs sels d'addition à un acide minéral ou organique, pharmaceutiquement acceptable.

La présente invention a également pour objet un procédé de préparation de composés de formule générale I, caractérisé en ce que:

soit

l'on condense

ou bien

un composé de formule générale II:

$$R\text{-}(CH_2)_m\text{-}X \qquad \textbf{(II)}$$

dans laquelle R et m ont la même signification que pour la formule I et X représente un groupe partant tel qu'un atome d'halogène, un radical mésyle ou un radical tosyle,

avec une amine de formule générale III

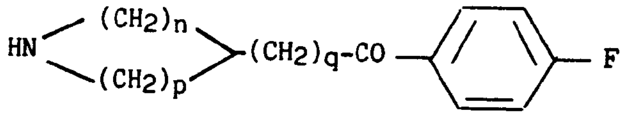 (III)

dans laquelle n, p, et q ont la signification indiquée ci-dessus pour la formule I,
ou bien
que l'on condense un composé de formule générale IV:

RH (IV)

dans laquelle R a la même signification que pour la formule I, avec un composé de formule V:

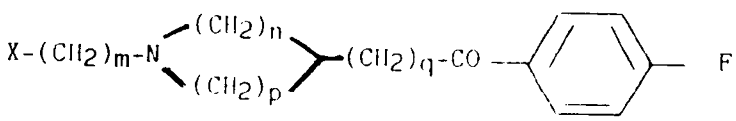 (V)

dans laquelle la signification de m, n, p, et q est identique à celle donnée pour la formule I et X représente un groupe partant dont la définition est identique à celle donnée pour la formule générale II, pour former les composés de formule I,
soit:
l'on cyclise un dérivé de l'amino-4 imidazole avec un composé de formule VI:

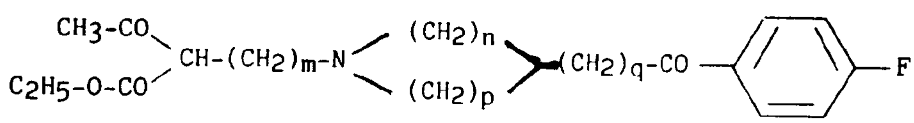 (VI)

dans laquelle m, n, p, et q ont la signification donnée pour la formule I, pour former les composés de la formule I,
dans laquelle R représente un radical de formule B et m, n, p et q ont la signification indiquée pour la formule I,
lesquels ensuite
si l'on désire, sont séparés en leurs stéréoisomères possibles ou/et salifiés par un acide organique ou minéral, pharmaceutiquement acceptable, pour former les sels d'addition correspondants.

Les composés de formule générale IV, quand R représente un groupement de formule A, sont soit des produits commerciaux, soit de préparation déjà connue. (Demande de Brevet EP 262 993).

Les composés de formule générale II, quand R représente un groupement de formule A, sont obtenus en traitant les composés de formule générale IV correspondants,

soit avec un bromochloroalcane de formule VII:

$Br(CH_2)_mCl$ (VII)

dans laquelle m a la même signification que pour la formule I,
soit avec une halohydrine de formule VIII:

$Hal(CH_2)_mOH$ (VIII)

dans laquelle m a la même signification que pour la formule I et Hal représente un atome d'halogène. Les alcools ainsi obtenus sont ensuite transformés en dérivés de formule II par des méthodes classiques.

Les composés de formule générale II, quand R représente un groupement de formule B et m est égal à 2, sont obtenus suivant la méthode décrite dans J.Heterocycl.Chem.,1974,11,p.873, par condensation de l'amino-4 carbamoyl-5 imidazole sur l'acétyl-3 dihydro-3H-furannone-2. L'alcool issu de cette réaction est ensuite transformé en dérivé chloré par action d'oxychlorure de phosphore. Ce traitement transforme également le radical carbamoyle ($R_2$) en nitrile.

La (chloro-2 éthyl)-3 tétrahydro-1,2,3,4 quinazoline dione-2,4 est un produit commercial (Janssen ®).

Quand R représente un groupement benzhydryloxy, les composés de formule II sont obtenus selon le procédé de synthèse décrit dans Organic Synthesis Collect.,Vol.IV,Wiley Ed, N.Y.,1963,p.72 ou selon la méthode

décrite dans J.Med. Chem.,1980,23,p.149.

Les composés de formule II, quand R représente un groupement de formule C, sont obtenus en suivant la méthode décrite dans J.Med.Chem.,1965,8, p.446.

Certaines amines de formule générale III sont déjà décrites dans la littérature (Brevet EP 13 612 et Chem.Pharm.Bull.,1977,25,p.1911).

L'amine de formule III, quand n est égal à 3, p égal à 1, et q égal à 1, peut être aussi préparée à partir de la chlorométhyl-3 méthyl-1 pipéridine et du fluoro-4 benzonitrile. Le composé issu de cette réaction est ensuite déméthylé selon des procédés connus pour donner l'amine secondaire attendue.

L'amine de formule III, quand n est égal à 3, p égal à 2, et q égal à zéro, est préparée à partir du N,N diméthyl amino-4 butyronitrile. Ce composé est condensé avec le chloro-3 iodo-1 propane pour former le (N,N diméthyl amino-2 éthyl)-2 chloro-5 pentanenitrile qui, après cyclisation, donne la cyano-4 méthyl-1 perhydroazépine. A partir de ce composé et en utilisant des méthodes classiques, on obtient l'amine attendue.

Le composé de formule III, quand n et p sont égals à 1 et q est égal à 1 est préparé à partir du cyanoacétate d'éthyle et de la chloro-2 fluoro-4 acétophénone. Le (fluoro-4 phénacyl)-2 cyanoacétate d'éthyle ainsi obtenu est ensuite mis en réaction avec l'éthylène glycol anhydre pour obtenir le [(cyano-2 éthoxycarbonyl)-2 éthyl]-2 (fluoro-4 phényl)-2 dioxolanne-1,3. L'hydrogénation de ce dernier composé conduit à l'[(aminométhyl-2 éthoxycarbonyl)-2 éthyl]-2 (fluoro-4 phényl)-2 dioxolanne-1,3, qu'on fait réagir avec l'iodure du méthylmagnésium pour obtenir la (fluoro-4 phényl)-2 [(oxo-2 azétidin-3-yl) méthyl]-2 dioxolanne. Ce composé est soumis à l'action d'hydrure de lithium et d'aluminium pour obtenir le composé attendu.

Les composés de formule V sont obtenus en traitant les amines de formule III soit avec un bromochloroalcane de formule VII, soit avec une halohydrine de formule VIII. Dans ce dernier cas, les alcools obtenus sont transformés en dérivés de formule V par des méthodes classiques.

Les composés de formule VI sont préparés par condensation des composés de formule V sur l'acétoacétate d'éthyle (Ann.Rep.Sankyo Res.lab.,(1977), 29,p.75-98).

La condensation des composés de formule III ou IV avec les composés II ou V est réalisée dans un solvant organique polaire en présence de sels minéraux tels que le carbonate de sodium et l'iodure de sodium à une température comprise entre 40°C et 120°C.

La cyclisation des dérivés d'amino-4 imidazole avec les composés de formule VI s'effectue à chaud et en présence d'acide phosphorique.

Parmi les acides pharmaceutiquement acceptables pour la préparation des sels d'addition aux composés de formule générale I, on peut citer les acides chlorhydrique, phosphorique, fumarique, citrique, oxalique, sulfurique, ascorbique, tartrique, maléïque, mandélique, méthanesulfonique etc...

Les composés de la présente invention possèdent des propriétés pharmacologiques fort intéressantes. Des essais pharmacologiques ont démontré leurs activités antagonistes au niveau des récepteurs 5-$HT_2$ et $\alpha_1$. Certains composés ayant de puissantes propriétés antisérotonine, et en particulier la kétansérine, possèdent des activités antiagrégante plaquettaire, antivasospastique et vasoprotectrice du plus grand intérêt. Toutefois, ces composés sont dépourvus d'activité $\alpha_1$-antagoniste. Les propriétés 5-$HT_2$ alliées à des propriétés $\alpha_1$-antagonistes, rendent les composés de l'invention particulièrement utiles dans le traitement de l'hypertension ou des affections secondaires à l'hypertension tout en assurant la protection de la paroi vasculaire.

Les composés de l'invention inhibent aussi d'une manière puissante les symptômes complexes et caractéristiques induites chez l'animal par l'injection de 5-hydroxy tryptophane. Les composés de la présente invention sont donc également antagonistes de la sérotonine au niveau des récepteurs de type 5-$HT_1$ (J.Ph.Ex-.Ther.(1984)228,N°1,p.133-139). Grâce à leurs propriétés antagonistes des récepteurs à la sérotonine au niveau central et tout particulièrement des récepteurs 5-$HT_2$ et 5-$HT_1$, les composés de l'invention peuvent être utilisés pour lutter contre certains effets indésirables de ces médiateurs. Ils trouvent donc leurs applications plus spécialement dans l'anxiété et la dysthymie (Ceulemans D.L.S., Hoppenbrouwers M.L., Gelders Y.G., Reyntjens A.K.M., Pharmacopsychiat.,(1985)18,p.303-305 et Le Bars, Neuronal Serotonin Eds Osborne. N.N and Hamon M., John Wiley and Sons Ltd, N.Y.,(1988), p.171-229), dans la dépression et le stress (Anisman H. and Zacharko R.M., Behav.Brain. Scienc.,(1982),5,p.89-137 et Blier P., de Montigny C. and Chaput Y., J-.Clin.Psychopharmacol.,(1987),7,p.245-335), le traitement de la douleur (Jacobs B.L. and Trulson M.E., TINS,(1979),Novem.,p.276-280), les troubles de mémoire (Markianos M., Hadjikonstantinou and Bistolaki E., Acta Neurol.Scand., (1982),66,p.267-275), la maladie de Parkinson (Le Bars, Neuronal Serotonin Eds. Osborne NN and Hamon M, John Wiley and Sons Ltd N.Y.,(1988),p.171-229), et la schizophrénie (Borison R.L., Havdala H.S. and Diamond B.I., Comms. Psychopharmacol.,(1978),2,p.209-214 et Iversen S.D., Neuropharmacol.,(1984),23,p.1553-1560).

Les composés de l'invention possèdent aussi des propriétés antihistaminiques. Ils peuvent donc être utilisés comme agents anti-allergiques, antiprurigineux pour le traitement des voies respiratoires telles que rhinites, rhumes de foins, pour le traitement de l'asthme, et d'oedème de Quincke.

L'invention s'étend aussi aux compositions pharmaceutiques contenant comme principe actif au moins un composé de formule générale I, ou l'un de ses sels avec un acide minéral ou organique pharmaceutiquement compatible, en association avec un ou plusieurs excipients inertes et appropriés.

Les compositions pharmaceutiques ainsi obtenues sont présentées avantageusement sous des formes diverses telles que par exemple comprimés, dragées, gélules, suppositoires, solutions injectables ou buvables.

La posologie peut varier largement en fonction de l'âge, du poids du patient, de la nature et de la sévérité de l'affection ainsi que de la voie d'administration. D'une manière générale, la posologie unitaire s'échelonnera entre 0,5 et 100 mg et la posologie journalière, utilisable en thérapeutique humaine, entre 0,5 mg et 300 mg.

La voie d'administration préférée est la voie orale ou parentérale.

Les exemples suivants, donnés à titre non-limitatif, illustrent l'invention.

Les points de fusion ont été mesurés selon la technique Micro-Köfler.

Les spectres de résonance magnétique nucléaire du proton (RMN-$^{1}$H) ou de carbone $^{13}$C (RMN-$^{13}$C) des composés de formule générale I ont été enregistrés selon le cas à 60, 200 et 400 MHz et sont indiqués dans le Tableau I.

**EXEMPLE 1**

**Chlorhydrate de la cyano-8 {[(fluoro-4 benzoyl)-4 pipéridino]-2 éthyl}-3 hydroxy-4 méthyl-2 imidazo [1,5-a] pyrimidine**

STADE A

Carbamoyl-8 hydroxy-4 (hydroxy-2 éthyl)-3 méthyl-2 imidazo [1,5-a] pyrimidine

Dans un tricol, introduire successivement 3 g de chlorhydrate d'amino-4 carbamoyl-5 imidazole, 1,51 g d'acétate de sodium, 1,69 g d'éthanol, 7 g d'acétyl-3 tétrahydrofurannone-2 et 45 ml de toluène. Chauffer 90 heures à reflux. Après refroidissement, reprendre le précipité formé par l'éthanol bouillant. Filtrer et reprendre le résidu dans l'eau bouillante, filtrer, laver à l'éthanol le résidu et ensuite sécher.

Rendement : 62%

Spectre de résonance magnétique nucléaire du proton (400 MHz, solvant DMSO-$d_6$) : 2,5 ppm,s,3H; 2,6 ppm,t,2H; 3,5 ppm,q,2H; 4,6 ppm,t,1H; 7,1-7,4 ppm,m,2H; 8,1 ppm,s,1H; 11,4 ppm,m,1H

STADE B

(Chloro-2 éthyl)-3 cyano-8 hydroxy-4 méthyl-2 imidazo [1,5-a] pyrimidine

Porter à 85°C, pendant une heure et 30 minutes, 0,17 mole du composé obtenu au Stade A en solution dans 200 ml d'oxychlorure de phosphore. Eliminer ensuite ce dernier par évaporation sous vide. Ajouter 100 ml d'eau et ajuster le pH à 7 à l'aide du bicarbonate de sodium pour cristalliser le composé attendu. Filtrer.

Rendement : 88%

Point de fusion : 225°C

Spectre de résonance magnétique nucléaire du proton (60 MHz, solvant DMSO-$d_6$): 2,45 ppm,s,3H; 2,9 ppm,t,2H; 3,7 ppm,t,2H; 8,15 ppm,s,1H; 13,0-13,4 ppm,1H échangeable

STADE C

Porter à reflux 9,7 g du composé obtenu au stade précédent, 9,5 g de (fluoro-4 benzoyl)-4 pipéridine, 23 g de carbonate de sodium, 0,5 g d'iodure de potassium et 800 ml de méthyl-4 pentanone-2. Laisser réagir pendant environ 15 heures, puis hydrolyser à chaud par 200 ml d'eau pendant 3 heures. Filtrer à chaud et rincer le précipité à l'acétone puis à l'éthanol.

Purifier sur colonne chromatographique contenant 300 g de silice 70-230 mesh en utilisant comme éluant un mélange de dichlorométhane et d'éthanol (90:10 V/V). Cristalliser le produit purifié dans un mélange de dichlorométhane et de méthanol (99:1 V/V). Former ensuite le chlorhydrate de la cyano-8 {[(fluoro-4 benzoyl)-4 pipéridino]-2 éthyl}-3 hydroxy-4 méthyl-2 imidazo [1,5-a] pyrimidine à l'aide de l'éthanol chlorhydrique.

Rendement : 35%

Point de fusion :> 260°C

**EXEMPLE 2**

**Chlorhydrate de la {[(fluoro-4 benzoyl)-4 pipéridino]-3 propyl}-1 (fluoro-2 benzyl)-4 pipérazine dione-2,6**

Porter à reflux pendant 5 heures un mélange de 8 g de (chloro-3 propyl)-1 (fluoro-2 benzyl)-4 pipérazine dione-2,6, 6,1 g de (fluoro-4 benzoyl)-4 pipéridine, 15 g de carbonate de sodium, 0,1 g d'iodure de potassium et de 250 ml de méthyl-4 pentanone-2.

Après concentration, le résidu est repris dans l'eau et extrait au benzène. L'huile ainsi obtenue est purifiée par chromatographie sur colonne sur 350 g de silice 70-230 mesh, en utilisant comme éluant le dichlorométhane puis un gradient 1-5% de méthanol. Former ensuite le chlorhydrate de la {[(fluoro-4 benzoyl)-4 pipéridino]-3 propyl}-1 (fluoro-2 benzyl)-4 pipérazine dione-2,6 en salifiant la base obtenue à l'aide de l'éthanol chlorhydrique.

Rendement : 42%

Point de fusion : 238°C

**EXEMPLE 3**

**Chlorhydrate de la {[(fluoro-4 benzoyl)-4 pipéridino]-3 propyl}-1 (pyridyl-2 méthyl)-4 pipérazine dione-2,6**

STADE A

(chloro-3 propyl)-1 (fluoro-4 benzoyl)-4 pipéridine

A un mélange de 5 g de (fluoro-4 benzoyl)-4 pipéridine et de 3,95 ml de triéthylamine dans 60 ml de diméthylformamide anhydre, ajouter à 20°C, goutte à goutte, 2,7 ml de chloro-1 iodo-3 propane en solution dans 10 ml de diméthylformamide. Maintenir l'agitation pendant 20 heures à 20°C, puis concentrer sous vide. Reprendre le résidu dans l'eau alcalinisée (pH=9-10) et extraire au dichlorométhane. L'huile obtenue après évaporation du solvant organique est purifiée sur colonne de silice (100 g ; 70-230 mesh) en utilisant comme éluant un mélange de dichlorométhane et de méthanol (99:1 V/V).

Rendement : 60%

Spectre de résonance magnétique nucléaire du proton (60 MHz, solvant $CDCl_3$): 1,6-3,4 ppm,m,13H; 3,6 ppm,t,2H; 7,1 ppm,t,2H; 7,8-8,1 ppm,dd,2H

STADE B

A une suspension de 0,36 g d'hydrure de sodium, ajouter à 20°C, une solution de 1,86 g (pyridyl-2 méthyl)-4 pipérazine dione-2,6 dans 30 ml de diméthylformamide. Porter à 60°C durant 30 minutes puis ajouter, à nouveau à 20°C, 2,7 g de (chloro-3 propyl)-1 (fluoro-4 benzoyl)-4 pipéridine en solution dans 30 ml de diméthylformamide. Maintenir l'agitation à 20°C environ 15 heures et compléter la réaction en portant 2 heures à 70°C. Après concentration, reprendre le résidu dans l'eau et extraire au benzène. Purifier l'huile obtenue par chromatographie sous pression sur 280 g de silice 230-400 mesh en éluant par un mélange de dichlorométhane et de méthanol (93,5:6,5 V/V). La base obtenue est transformée en chlorhydrate dans l'éthanol. Le précipité obtenu est ensuite filtré.

Rendement : 35%

Point de fusion : 192°C

**EXEMPLE 4**

**Chlorhydrate de la {[(fluoro-4 benzoyl)-4 pipéridino]-4 butyl}-1 (fluoro-2 benzyl)-4 pipérazine dione-2,6**

Ajouter goutte à goutte à 20°C, 2,61 g de (fluoro-2 benzyl)-4 (bromo-4 butyl)-1 pipérazine dione-2,6 en solution dans 40 ml de diméthylformamide, à un mélange de 1,4 g de (fluoro-4 benzoyl)-4 pipéridine et de 0,84 g de triéthylamine dans 50 ml de diméthylformamide. Après 15 heures d'agitation à 20°C, concentrer le solvant sous vide. Reprendre le résidu dans l'eau et extraire au dichlorométhane. Purifier l'huile obtenue par chromatographie sur 210 g de silice 230-400 mesh en éluant avec un mélange de dichlorométhane et de méthanol

(95:5 V/V).

La base ainsi obtenue est ensuite salifiée par l'éthanol chlorhydrique dans un mélange d'éthanol et d'éther éthylique.
Rendement : 40%
Point de fusion: 198°C

**EXEMPLE 5**

**Tartrate de (fluoro-2 benzyl)-4 {[(fluoro-4 phénacyl)-3 pyrrolidinyl-1]-3 propyl}-1 pipérazine dione-2,6**

Chauffer 3 heures à 120°C un mélange de 2,5 g de (fluoro-4 phénacyl)-3 pyrrolidine de 4 g de (chloro-3 propyl)-1 (fluoro-2 benzyl)-4 pipérazine dione-2,6 de 1,3 g de carbonate de sodium, de 0,1 g d'iodure de potassium et de 70 ml de méthyl-4 pentanone-2.

Concentrer, reprendre à l'eau, extraire au dichlorométhane, puis sécher la phase organique, concentrer.

Purifier l'huile obtenue par chromatographie sur 300 g de silice 60 (70-230 mesh) en utilisant comme éluant un mélange de dichlorométhane et de méthanol (98:2 V/V).

On obtient 2 g d'une huile. Pour obtenir le monotartrate, on salifie dans un mélange d'éthanol et d'éther éthylique.
Rendement : 45%
Point de fusion : ≃ 65°C

**EXEMPLE 6**

**Ditartrate de la (fluoro-2 benzyl)-4 {[(fluoro-4 phénacyl)-3 pipéridino]-3 propyl}-1 pipérazine dione-2,6**

STADE A

(fluoro-4 phénacyl)-3 méthyl-1 pipéridine

A 5,55 g de magnésium en suspension dans 25 ml de tétrahydrofuranne, ajouter sous azote une solution de 33,7 g de chlorométhyl-3 N-méthyl pipéridine dans 80 ml de tétrahydrofuranne. Porter le mélange 2 heures à reflux, puis refroidir à 10°C et y ajouter une solution contenant 30,5 g de fluoro-4 benzonitrile dissous dans 100 ml de tétrahydrofuranne. Porter pendant 3 heures à 40°C, puis hydrolyser avec une solution de 68 ml d'acide chlorhydrique (d=1,18) dans 42 ml d'eau. Chauffer 1 heure à reflux, refroidir, extraire à l'éther éthylique. Après élimination de la phase organique, alcaliniser la phase aqueuse (pH=9) à l'aide de la lessive de soude, filtrer sur célite, extraire la phase aqueuse au dichlorométhane, sécher sur sulfate de sodium et concentrer pour obtenir une huile.
Rendement : 85%

Spectre de résonance magnétique nucléaire du proton (60 MHz, solvant $CDCl_3$) : 1,3-2,5 ppm,m,7H; 2,25 ppm,s,3H; 2,75 ppm,m,2H; 2,90 ppm,d,2H; 7,15 ppm,m,2H; 8,0 ppm,m,2H

STADE B

Ethoxycarbonyl-1 (fluoro-4 phénacyl)-3 pipéridine

Porter 5 heures à reflux un mélange de 44,5 g de l'huile obtenue au Stade A, 82 g (0,755 mole) de chloroformiate d'éthyle, 20 g de carbonate de sodium, 1 g d'iodure de potassium et 400 ml de toluène. Refroidir ensuite le mélange, y ajouter 200 ml d'acide chlorhydrique 1N, décanter la phase organique. Laver la phase organique à l'eau, puis sécher cette phase sur sulfate de sodium anhydre. Concentrer. L'huile obtenue est utilisée à l'étape suivante.
Rendement : 85%

STADE C

(fluoro-4 phénacyl)-3 pipéridine

Porter pendant 2 heures 30 minutes à reflux toute l'huile obtenue dans l'étape B avec 250 ml d'acide bromohydrique à 48% (d=1,48). Concentrer ensuite sous vide, amener le résidu à pH 10-11 à l'aide de lessive de

soude, extraire au dichlorométhane, sécher sur sulfate de sodium et concentrer.
Rendement : 85%

STADE D

Porter pendant 4 heures à 120°C un mélange de 2 g de (fluoro-4 phénacyl)-3 pipéridine, de 2,7 g de (chloro-3 propyl)-1 (fluoro-2 benzyl)-4 pipéridine dione-2,6, 1 g de carbonate de sodium, 0,1 g d'iodure de potassium et 30 ml de méthyl-4 pentanone-2.

Concentrer ensuite l'ensemble, reprendre le résidu dans 50 ml d'eau, extraire au dichlorométhane et concentrer.

Soumettre l'huile obtenue à une purification sur colonne chromatographique contenant 250 g de silice 60 (70-230 mesh) en utilisant comme éluant un mélange de dichlorométhane et de méthanol. (98:2 V/V).

Salifier la (fluoro-2 benzyl)-4 {[(fluoro-4 phénacyl)-3 pipéridino]-3 propyl}-1 pipérazine dione-2,6 obtenue en ajoutant deux équivalents d'acide tartrique à une solution éthanolique du produit. Concentrer et reprendre le précipité dans l'éther éthylique.
Rendement : 45%
Point de fusion : 75°C

**EXEMPLE 7**

**Dichlorhydrate de la {[(fluoro-4 benzoyl)-4 pipéridino]-3 propyl}-1 (pyrimidinyl-2)-4 pipérazine**

Agiter pendant 24 heures à température ambiante un mélange de 5 g de (chloro-3 propyl)-1 (fluoro-4 benzoyl)-4 pipéridine et de 3,2 g de (pyrimidinyl-2)-4 pipérazine en présence d'un excès de carbonate de sodium dans 70 ml de diméthylformamide.

Eliminer le précipité formé. Concentrer la solution et reprendre le résidu dans 100 ml d'eau. Extraire cette phase aqueuse trois fois avec 100 ml de dichlorométhane. Sécher la phase organique sur sulfate de sodium anhydre et ensuite concentrer pour obtenir un résidu huileux qui cristallise dans l'éther isopropylique.
Rendement : 25%

Le dichlorhydrate de la {[(fluoro-4 benzoyl)-4 pipéridino]-3 propyl}-1 (pyrimidinyl-2)-4 pipérazine est obtenu après addition d'une quantité adéquate d'éthanol chlorhydrique.
Point de fusion : 260°C

**EXEMPLE 8**

**Chlorhydrate de la carbamoyl-8 {[(fluoro-4 benzoyl)-4 pipéridino]-2 éthyl}-3 hydroxy-4 méthyl-2 imidazo [1,5-a] pyrimidine**

STADE A

(chloro-2 éthyl)-1 (fluoro-4 benzoyl)-4 pipéridine

Additionner à -10°C, 9,56 g d'oxyde d'éthylène à une solution de 45 g de (fluoro-4 benzoyl)-4 pipéridine dans 500 ml de méthanol anhydre. Après 15 heures d'agitation à 20°C puis 5 heures à 50°C, la réaction est pratiquement terminée. Concentrer sous vide. Purifier le résidu obtenu sur colonne chromatographique contenant 100 g de silice 70-230 mesh en utilisant comme éluant le dichlorométhane.

L'huile obtenue est mise en solution dans 800 ml de benzène anhydre. A cette solution, ajouter à 5°C, goutte à goutte, 10,4 ml de chlorure de thionyle. Porter ensuite pendant 2 heures 30 minutes à reflux. Après refroidissement, filtrer le précipité. Reprendre celui-ci dans l'eau alcalinisée et extraire au benzène. Après évaporation, le résidu obtenu est purifié par passage sur 100 g de silice 70-230 mesh, en utilisant comme éluant le dichlorométhane.
Rendement : 41%

Spectre de résonance magnétique nucléaire du proton (60 MHz, solvant $CDCl_3$) : 1,6-3,5 pppm,m,11H; 3,65 ppm,t,2H; 7-7,5 ppm,m,2H; 7,8-8,3 ppm, m,2H

STADE B

Acétyl-2 [(fluoro-4 benzoyl)-4 pipéridino]-4 butyrate d'éthyle

A une suspension de 1,68 g d'hydrure de sodium à 60% dans l'huile dans 100 ml de tétrahydrofuranne anhydre, ajouter à 0°C une solution de 5,45 g d'acétoacétate d'éthyle dans 20 ml de tétrahydrofuranne. Maintenir le milieu à 20°C pendant 1 heure 30 minutes. Ajouter 6,3 g d'iodure de sodium puis additionner à 0°C, 11,3 g de (chloro-2 éthyl)-1 (fluoro-4 benzoyl)-4 pipéridine dans 100 ml de tétrahydrofuranne. Porter le mélange réactionnel à reflux pendant 15 heures. Concentrer sous vide. Reprendre le résidu dans l'eau, extraire au dichlorométhane.

Après évaporation, l'huile est purifiée sur 300 g de silice 70-230 mesh en utilisant comme éluant un mélange de dichlorométhane et de méthanol (99:1 V/V).

Rendement : 48%

Spectre de résonance magnétique nucléaire du proton (200 MHz, solvant $CDCl_3$) : 1,3 ppm,t,3H; 1,5-2,5 ppm,t+s+m,2H+3H+9H; 2,8-3,0 ppm,t,1H; 4,1-4,3 ppm,q,2H; 7-7,2 ppm,t,2H; 7,9-8 ppm,dd,2H

STADE C

A 20°C, préparer un mélange homogène contenant 2,5 g de chlorhydrate d'amino-4 carbamoyl-5 imidazole, 6,15 g du composé obtenu au stade précédent et 15 g d'acide phosphorique. Porter ce mélange à 80°C pendant environ 30 minutes, puis hydrolyser par de la glace. Filtrer le précipité obtenu. Salifier à l'aide d'un excès d'éthanol chlorhydrique en portant à reflux dans un mélange hydroalcoolique (Ethanol 800 ml+$H_2O$ 60 ml). Filtrer après refroidissement.

Rendement : 72%

Point de fusion : >300°C

**EXEMPLE 9**

**Chlorhydrate de la carbamoyl-8 {[(fluoro-4 phénacyl)-3 pyrrolidinyl-1]-2 éthyl}-3 hydroxy-4 méthyl-2 imidazo [1,5-a] pyrimidine**

STADE A

(fluoro-4 phénacyl)-3 (hydroxy-2 éthyl)-1 pyrrolidine

Chauffer 6 heures à reflux un mélange de 30,5 g de (fluoro-4 phenacyl)-3 pyrrolidine (Chem.Pharm.Bull., 1977,25(8), p.1911-1922), 19,4 g de bromo-2 éthanol, 15,6 g de carbonate de sodium et 400 ml d'acétonitrile. Concentrer le tout, reprendre le résidu dans 150 ml d'eau salée, extraire 5 fois par 150 ml de dichlorométhane, sécher cette phase organique sur sulfate de sodium anhydre et concentrer. L'huile obtenue est purifiée par chromatographie sur 650 g de silice (70-230 mesh) en utilisant comme éluant un mélange de dichlorométhane, de méthanol et d'ammoniaque (96:4:0,4 V/V). On obtient une huile incolore.

Rendement : 50%

Spectre de résonance magnétique nucléaire du proton (60 MHz, solvant $CDCl_3$) : 1,8-2,5 ppm,m,2H; 2,9-4,0 ppm,m,11H; 7,2 ppm,t,2H; 8 ppm,dd,2H

STADE B

Acétyl-2 [(fluoro-4 phénacyl)-3 pyrrolidinyl-1]-4 butyrate d'éthyle

A une suspension de 8,5 g du produit obtenu au stade A dans 100 ml de tétrahydrofuranne, additionner à 0°C une solution de 3,88 g de chlorure de l'acide méthanesulfonique. Laisser agir 3 heures à 15°C, puis ajouter cette suspension à une solution refroidie à 0°C de 4,4 g d'acétoacétate d'éthyle contenant 1,63 g d'hydrure de sodium dans 100 ml de tétrahydrofuranne. Porter ensuite 12 heures à reflux, hydrolyser avec 5 ml d'eau, concentrer l'ensemble, reprendre le résidu dans 150 ml de dichlorométhane. Laver la phase organique avec 50 ml d'eau, sécher sur sulfate de sodium anhydre et concentrer. Purifier l'huile obtenue par chromatographie sur 600 g de silice 70-230 mesh en utilisant comme éluant un mélange de dichlorométhane et de méthanol (99:1 V/V).

Rendement : 50%

Spectre de résonance magnétique nucléaire du proton (60 MHz, solvant $CDCl_3$) : 1,1-1,4 ppm,t,3H; 1,4-3,2 ppm,m,11H; 2,25 ppm,s,3H; 3,15 ppm, d,2H; 3,65 ppm,t,1H; 4,1-4,3 ppm,q,2H; 7,0-7,2 ppm,t,2H; 7,9-8,0 ppm,dd,2H

STADE C

Mélanger 7 g du composé obtenu au stade précédent, 3,13 g de chlorhydrate d'amino-4 carbamoyl-5 imidazole et 60 g d'acide phosphorique. Chauffer ce mélange 1 heure à 85°C, puis hydrolyser à 5°C avec 100 g de glace et 100 ml d'eau. Amener l'ensemble à pH 12 à l'aide de la lessive de soude et extraire 4 fois par du dichlorométhane. Concentrer la phase organique. Dissoudre le précipité formé et le concentrat de la phase organique dans 150 ml d'eau à pH 12 et extraire la solution obtenue au perforateur de Jalade à l'aide de dichlorométhane, pendant 24 heures. On obtient ainsi dans la phase organique, un précipité blanc, que l'on filtre.

Dissoudre ce précipité dans 180 ml d'éthanol à 60°C, y ajouter deux équivalents d'éthanol chlorhydrique, filtrer après 12 heures le précipité formé pour obtenir le chlorhydrate de la carbamoyl-8 {[(fluoro-4 phénacyl)-3 pyrrolidinyl-1]-2 éthyl}-3 hydroxy-4 méthyl-2 imidazo [1,5a] pyrimidine.
Rendement : 30%
Point de fusion : >270°C

**EXEMPLE 10**

**Chlorhydrate de la dioxo-2,4 {[(fluoro-4 phénacyl)-3 pyrrolidinyl-1]-2 éthyl}-3 tétrahydro-1,2,3,4 quinazoline**

Porter 12 heures à reflux un mélange de 5,5 g de (chloro-2 éthyl)-3 dioxo-2,4 tétrahydro-1,2,3,4 quinazoline, 4,2 g de (fluoro-4 phénacyl)-3 pyrrolidine, 5,1 g d'hydrogénocarbonate de sodium et 100 ml de toluène. Laver ensuite la phase organique à l'eau et concentrer le toluène sous vide. L'huile obtenue est ensuite purifiée par chromatographie sur 300 g de silice 70-230 mesh en utilisant comme éluant un mélange de dichlorométhane et de méthanol (97:3 V/V).

Le chlorhydrate correspondant est obtenu dans un mélange d'éther et d'acétone.
Rendement : 35%
Point de fusion : 145°C.

**EXEMPLE 11**

**Maléate du bis(fluoro-4 phényl)-1,1 [(fluoro-4 benzoyl)-4 pipéridino]-4 oxy-2 butane**

STADE A

Bis (fluoro-4 phényl)-1,1 chloro-4 oxy-2 butane

Porter à reflux un mélange de 0,362 mole de difluoro-4,4′ benzhydrol de 0,77 mole de chloro-2 éthanol et de 0,257 mole d'acide para-toluène sulfonique dans 500 ml de toluène anhydre. L'eau formée est éliminée à l'aide d'un appareil Dean-Stark. Refroidir. Laver la phase organique par de l'eau, puis par de l'eau carbonatée, puis à nouveau par de l'eau.

Après concentration, on obtient une huile.
Rendement : 99%

Spectre de résonance magnétique nucléaire du proton (60 MHz, solvant $CDCl_3$): 3,7 ppm,s,4H; 5,4 ppm,s,1H; 6,9-7,6 ppm,m,8H

STADE B

Porter à reflux pendant 4 jours un mélange de 2,47 g du composé obtenu au stade précédent, 2,07 g de (fluoro-4 benzoyl)-4 pipéridine, 10,6 g de carbonate de sodium et de 100 ml de méthyl-4 pentanone-2. Après concentration, le résidu est repris dans le dichlorométhane et lavé à l'eau. L'huile obtenue est purifiée sur 150 g de silice (230-400 mesh) à l'aide d'un mélange de dichlorométhane et d'éthanol (99:1 V/V).

La base est ensuite transformée en maléate dans un mélange d'éther éthylique et d'acétone et le sel obtenu est recristallisé dans le même mélange.
Rendement : 35%

Point de fusion : 124°C

EXEMPLE 12

Dichlorhydrate du aza-2 (benzodioxanne-1,4 yl-2)-1 [(fluoro-4 benzoyl)-4 pipéridino]-5 pentane

Porter à reflux un mélange de 8,7 g d'amino méthyl-2 benzodioxanne-1,4 (préparé selon le procédé décrit dans J.Med.Chem.(1965),8,p.446), 5 g de (chloro-3 propyl)-1 (fluoro-4 benzoyl)-4 pipéridine, 1,86 g de carbonate de sodium, 50 ml d'acétonitrile et 60 ml de méthyl éthyl cétone pendant 48 heures.

Concentrer ensuite l'ensemble, y ajouter 150 ml d'eau, extraire au dichlorométhane, sécher la phase organique sur sulfate de sodium anhydre et concentrer. Les 12 g d'huile obtenue sont purifiés par chromatographie sur 400 g de silice 60 Merck ®(70-230 mesh) en utilisant comme éluant un mélange de dichlorométhane, de méthanol et d'ammoniaque (98:2:0,2 V/V).

4 g d'une huile sont ainsi récupérés, dont on fait le dichlorhydrate dans l'acétone.
Rendement : 50%
Point de fusion : 238°C

EXEMPLE 13

Chlorhydrate de la carbamoyl-8 {[(fluoro-4 phénacyl)-3 pipéridino]-2 éthyl}-3 hydroxy-4 méthyl-2 imidazo [1,5a] pyrimidine

STADE A

(fluoro-4 phénacyl)-3 (hydroxy-2 éthyl)-1 pipéridine

Porter à reflux pendant 12 heures un mélange de 28,5 g de (fluoro-4 phénacyl)-3 pipéridine obtenue dans le Stade C de l'exemple 6, 17,7 g de bromo-2 éthanol, 13,7 g du carbonate de sodium et 250 ml d'acétonitrile. Ensuite concentrer l'ensemble, reprendre le résidu dans 100 ml d'eau et extraire au dichlorométhane. Sécher la phase organique et la concentrer.
Rendement : 85%

Spectre de résonance magnétique nucléaire du proton (200 MHz, solvant $CDCl_3$) : 0,9-2,5 ppm,m,7H; 2,5 ppm,t,2H; 2,7-2,9 ppm,m,2H; 2,8-2,9 ppm,d,2H; 3,5-3,6 ppm,t,2H; 7,0-7,2 ppm,t,2H; 8,0 ppm,dd,2H

STADE B

(chloro-2 éthyl)-1 (fluoro-4 phénacyl)-3 pipéridine

Additionner à 5°C une solution de 15,1 g de chlorure de thionyle dans 20 ml de benzène à une solution de 30,5 g du produit obtenu au stade précédent dissous dans 200 ml de benzène anhydre. Porter ensuite 3 heures à reflux, puis concentrer le tout, reprendre le résidu dans 100 ml d'eau, extraire au benzène, sécher la phase organique et la concentrer. Purifier l'huile obtenue par filtration sur 250 g de silice (70-230 mesh) en utilisant comme éluant un mélange de dichlorométhane et de méthanol (98:2 V/V)
Rendement : 75%

STADE C

Acétyl-2 [(fluoro-4 phénacyl)-3 pipéridino]-4 butyrate d'éthyle

Ajouter à 0°C une solution de 6,2 g d'acétoacétate d'éthyle à une suspension de 1,13 g d'hydrure de sodium dans 150 ml de tétrahydrofuranne. Après 15 minutes à cette température, ajouter 7,1 g d'iodure de sodium anhydre puis à 0°C, 13,5 g du produit obtenu dans le Stade B. Porter ensuite le mélange à reflux pendant 12 heures, puis concentrer l'ensemble, reprendre le résidu dans l'eau et extraire au dichlorométhane. Purifier les 16,9 g d'huile obtenue par chromatographie sur 900 g de silice (70-230 mesh) en utilisant comme éluant un mélange de dichlorométhane et de méthanol (98:2 V/V).
Rendement : 45%

Spectre de résonance magnétique nucléaire du proton (200 MHz, solvant $CDCl_3$) : 1,3-2,5 pmm,m+s+m+m+m,2H+3H+2H+1H+6H; 2,6-3,0 ppm,m,4H; 3,5 ppm,t,1H; 4,15 ppm,q,2H; 7,15 ppm,t,2H; 8,0

ppm,dd,2H

STADE D

Dans un ballon muni d'un refrigérant, portant pendant 60 heures à reflux un mélange de 5 g du produit obtenu au stade précédent, 1,72 g de chlorhydrate d'amino-5 carbamoyl-4 imidazole, 0,87 g d'acétate de sodium anhydre et 200 ml d'éthanol. Filtrer ensuite le précipité formé.

Salifier cette base en la dissolvant dans de l'éthanol à chaud et y ajouter deux équivalents d'acide chlorhydrique éthanolique. Refroidir et filtrer le précipité obtenu pour isoler le chlorhydrate.
Rendement : 55%
Point de fusion : ≃270°C

**EXEMPLE 14**

**Chlorhydrate de la {[(fluoro-4 benzoyl)-4 pipéridino]-3 propyl}-1 (méthyl-2 benzyl)-4 pipérazine dione-2,6**

Ce composé a été préparé selon le procédé décrit dans l'exemple 3 en utilisant au Stade B la (méthyl-2 benzyl)-4 pipérazine dione-2,6 au lieu de la (pyridyl-2 méthyl)-4 pipérazine dione-2,6.
Rendement : 30%
Point de fusion : 249°C

**EXEMPLE 15**

**Chlorhydrate de la {[(fluoro-4 benzoyl)-4 pipéridino]-3 propyl}-1 (méthyl-3 benzyl)-4 pipérazine dione-2,6**

Ce composé a été préparé selon le procédé décrit dans l'exemple 3 en utilisant au Stade B la (méthyl-3 benzyl)-4 pipérazine dione-2,6 au lieu de la (pyridyl-2 méthyl)-4 pipérazine dione-2,6.
Rendement : 40%
Point de fusion : 231°C

**EXEMPLE 16**

**Chlorhydrate de la (chloro-4 benzyl)-4 {[(fluoro-4 benzoyl)-4 pipéridino]-3 propyl}-1 pipérazine dione-2,6**

Ce composé a été préparé selon le procédé décrit dans l'exemple 3 en utilisant au Stade B la (chloro-4 benzyl)-4 pipérazine dione-2,6 au lieu de la (pyridyl-2 méthyl)-4 pipérazine dione-2,6
Rendement : 30%
Point de fusion : 239°C

**EXEMPLE 17**

**Chlorhydrate de la {[(fluoro-4 benzoyl)-4 pipéridino]-2 éthyl}-1 (fluoro-2 benzyl)-4 pipérazine dione-2,6**

Ce composé a été préparé à partir de la (chloro-2 éthyl)-1 (fluoro-4 benzoyl)-4 pipéridine et de la (fluoro-2 benzyl)-4 pipérazine dione-2,6 selon le procédé décrit dans l'exemple 3, Stade B.
Rendement : 31%
Point de fusion : 197°C

**EXEMPLE 18**

**Chlorhydrate de la {[(fluoro-4 benzoyl)-4 pipéridino]-3 propyl}-1 (méthoxy-3 benzyl)-4 pipérazine dione-2,6**

Ce composé a été préparé selon le procédé décrit dans l'exemple 3 en utilisant au Stade B la (méthoxy-3 benzyl)-4 pipérazine dione-2,6 au lieu de la (pyridyl-2 méthyl)-4 pipérazine dione-2,6
Rendement : 45%
Point de fusion : 202°C

**EXEMPLE 19**

**Chlorhydrate de la [(chloro-4 phényl)phényl méthyl]-4 {[(fluoro-4 benzoyl)-4 pipéridino]-2 éthyl}-1 pipérazine dione-2,6**

Ce composé a été préparé à partir de la (chloro-2 éthyl)-1 (fluoro-4 benzoyl)-4 pipéridine et de la [(fluoro-4 phényl) phényl méthyl]-4 pipérazine dione-2,6 selon le procédé décrit dans l'exemple 3, Stade B.
Rendement : 55%
Point de fusion : 180°C

**EXEMPLE 20**

**Trichlorhydrate de la {[(fluoro-4 phénacyl)-3 pyrrolidinyl-1]-3 propyl}-1 (pyrimidinyl-2)-4 pipérazine**

Ce composé a été préparé selon le procédé décrit dans l'exemple 5 en utilisant la (chloro-3 propyl)-1 (pyrimidinyl-2)-4 pipérazine au lieu de la (chloro-3 propyl)-1 (fluoro-2 benzyl)-4 pipérazine dione-2,6.
Rendement : 45%

**EXEMPLE 21**

**Dichlorhydrate de la {[(fluoro-4 benzoyl)-4 pipéridino]-2 éthyl}-4 (pyrimidinyl-2)-1 pipérazine**

Ce composé a été préparé à partir de la (chloro-2 éthyl)-1 (fluoro-4 benzoyl)-4 pipéridine et de la (pyrimidinyl-2)-1 pipérazine selon le procédé décrit dans l'exemple 3, Stade B.
Rendement : 30%
Point de fusion : > 260°C

**EXEMPLE 22**

**Chlorhydrate de la carbamoyl-8 {[(fluoro-4 benzoyl)-4 pipéridino]-3 propyl}-3 hydroxy-4 méthyl-2 imidazo [1,5-a] pyrimidine**

Ce composé a été préparé selon le procédé décrit dans l'exemple 8 mais en utilisant au Stade B, la (chloro-3 propyl)-1 (fluoro-4 benzoyl)-4 pipéridine au lieu de la (chloro-2 éthyl)-1 (fluoro-4 benzoyl)-4 pipéridine.
Rendement : 60%
Point de fusion : > 260°C

**EXEMPLE 23**

**Maléate du [(fluoro-4 benzoyl)-4 pipéridino]-4 [(fluoro-4 phényl)-phényl]-1 oxa-2 butane**

Ce composé a été synthétisé selon le procédé décrit dans l'exemple 11 en utilisant au Stade A le fluoro-4 benzhydrol au lieu du difluoro-4,4′ benzhydrol.
Rendement : 38%
Point de fusion : 136°C

**EXEMPLE 24**

**Trichlorhydrate de la {[(fluoro-4 benzoyl)-4 pipéridino]-2 éthyl}-1 (pyridyl-2 méthyl)-4 pipérazine dione-2,6**

Ce composé a été préparé selon le procédé décrit dans l'exemple 3 Stade B mais en utilisant la (chloro-2 éthyl)-1 (fluoro-4 benzoyl)-4 pipéridine au lieu de la (chloro-3 propyl)-1 (fluoro-4 benzoyl)-4 pipéridine.
Rendement : 79%

Point de fusion : 160°C

**EXEMPLE 25**

**Maléate du [(fluoro-4 benzoyl)-4 pipéridino]-4 [(fluoro-2 phényl)phényl]-1 oxo-2 butane**

Ce composé a été synthétisé selon le procédé décrit dans l'exemple 11 en utilisant au Stade A le fluoro-2 benzhydrol au lieu du difluoro-4,4′ benzhydrol.
Rendement : 35%
Point de fusion : 132°C

**EXEMPLE 26**

**Maléate du [(fluoro-4 benzoyl)-4 pipéridino]-4 [(méthoxy-4 phényl)phényl]-1 oxo-2 butane**

Ce composé a été synthétisé selon le procédé décrit ci-dessus en utilisant le méthoxy-4 benzhydrol.
Rendement : 35%
Point de fusion : 132°C

**EXEMPLE 27**

**Chlorhydrate de la cyano-8 {[(fluoro-4 benzoyl)-4 perhydroazépinyl-1]-2 éthyl]-3 hydroxy-4 méthyl-2 imidazo [1,5-a] pyrimidine**

STADE A

N,N diméthyl amino-4 butyronitrile

A une solution chauffée à 80°C de 400 g de chloro-4 butyronitrile dans 1200 ml d'éthanol pur, ajouter 420 g de diméthylamine. Après 12 heures à reflux, concentrer le solvant, y ajouter ensuite 3 litres d'éther éthylique, et éliminer le précipité formé. Concentrer le filtrat et le distiller sous 12 mm Hg.

Spectre de résonance magnétique nucléaire du proton (200 MHz, solvant $CDCl_3$) : 1,75 ppm,q,2H; 2,25 ppm,s,6H; 2,3-2,5 ppm,t+t,2H+2H

STADE B

[(N,N diméthylamino)-2 éthyl]-2 chloro-5 pentanenitrile

Ajouter une solution de 0,800 mole du produit obtenu au stade précédent à une solution refroidie à -85°C de 0,800 mole de diisopropylamidure de lithium dans 600 ml de tétrahydrofuranne. Laisser 20 minutes à -85°C, puis y additionner 0,800 mole de chloro-3 iodo-1 propane pur en 5 minutes. Laisser agiter pendant une heure puis hydrolyser à -80°C avec 500 ml d'acide acétique à 3%. Concentrer sous vide, reprendre dans 250 ml d'eau, extraire au dichlorométhane, concentrer cet extrait.
Rendement : 90%

Spectre de résonance magnétique nucléaire du proton (200 MHz, solvant $CDCl_3$) : 1,6-2,1 ppm,m,6H; 2,2 ppm,s,6H; 2,3-2,6 ppm,m,2H; 2,75 ppm,m,1H; 3,6 ppm,t,2H

STADE C

Cyano-4 méthyl-1 perhydroazépine

Porter 12 heures à 120°C un mélange de 140 g du produit précédent et de 850 ml de nitrobenzène, puis refroidir à 15°C et y ajouter 2 litres d'éther éthylique. Filtrer le précipité obtenu et le rincer à l'éther. Mélanger ensuite le produit obtenu avec un litre de décanol et porter l'ensemble à reflux pendant deux heures. Refroidir ensuite, extraire quatre fois avec 500 ml d'acide chlorhydrique 1N, neutraliser cet extrait à la soude et l'extraire au dichlorométhane. Distiller l'huile sous vide.
Rendement : 70%

Spectre de résonance magnétique nucléaire du proton (200 MHz, solvant $CDCl_3$) : 1,5-2,2 ppm,m,6H; 2,35

ppm,s,3H; 2,5-3,0 ppm,m+m,1H+4H

STADE D

(fluoro-4 benzoyl)-4 méthyl-1 perhydroazépine

A une solution de 0,143 mole de bromure de fluoro-4 phényl magnésium dans 150 ml d'éther éthylique, ajouter à 20°C une solution de 0,0725 mole de l'amine obtenue au Stade C. Porter pendant 5 heures à reflux, laisser ensuite le milieu réactionnel 12 heures à température ambiante, puis hydrolyser avec 40 ml d'acide chlorhydrique concentré et 25 ml d'eau. Porter pendant 1 heure 30 à reflux, refroidir, décanter l'éther, basifier la phase aqueuse et l'extrait au dichlorométhane. On purifie l'huile obtenue par cristallisation de l'oxalate correspondant.
Rendement : 60%
Spectre de résonance magnétique nucléaire du proton (200 MHz, solvant $CDCl_3$) : 1,65-2,20 ppm,m,6H; 2,5-2,8 ppm,m,4H; 3,4 ppm,s,3H; 3,6 ppm,m,1H; 7,1 ppm,t,2H; 7,95 ppm,dd,2H

STADE E

(fluoro-4 benzoyl)-4 éthoxycarbonyl-1 perhydroazépine

La carbamoylation de l'amine obtenue dans le Stade C s'effectue suivant le mode opératoire décrit dans l'étape B de l'exemple 6. Le composé obtenu est ensuite purifié par chromatographie sur colonne.
Rendement : 65%
Spectre de résonance magnétique nucléaire du proton (200 MHz, solvant $CDCl_3$) : 1,25 ppm,t,3H; 1,5-2,2 ppm,m,6H, 3,05-3,9 ppm,m,5H; 4,15 ppm, q,2H; 7,1 ppm,t,2H; 7,9-8,1 ppm,dd,2H

STADE F

(fluoro-4 benzoyl)-4 perhydroazépine

Ce composé a été obtenu à partir de la (fluoro-4 benzoyl)-4 éthoxycarbonyl-1 perhydroazépine à l'aide d'acide bromhydrique suivant le mode opératoire décrit dans le Stade C de l'exemple 6. L'amine obtenue est utilisée au Stade G sans purification.

STADE G

Porter à reflux le composé obtenu au Stade F, 9,7 g de (chloro-2 éthyl)-3 cyano-8 hydroxy-4 méthyl-2 imidazo [1,5a] pyrimidine, 23 g de carbonate de sodium, 0,5 g d'iodure de potassium et 800 ml de méthyl-4 pentanone-2. Procéder ensuite selon la méthode décrite dans l'exemple 1 Stade C, pour obtenir le chlorhydrate attendu.
Rendement : 35%
Point de fusion : > 260°C

**EXEMPLE 28**

**Chlorhydrate de la cyano-8 {[(fluoro-4 phénacyl)-3 pyrrolidinyl-1]-2 éthyl}-3 hydroxy-4 méthyl-2 imidazo [1,5-a] pyrimidine**

Porter 3 heures à 85°C un mélange de 3,2 g de carbamoyl-8 {[(fluoro-4 phénacyl)-3 pyrrolidinyl-1]-2 éthyl}-3 hydroxy-4 méthyl-2 imidazo [1,5a] pyrimidine et 20 ml d'oxychlorure de phosphore. Concentrer ensuite sous vide, reprendre le résidu dans l'eau, neutraliser avec une solution de carbonate de sodium et extraire cinq fois avec du chloroforme. Le solide obtenu est purifié par chromatographie sur silice 70-230 mesh en utilisant comme éluant un mélange contenant du dichlorométhane, du méthanol et d'ammoniaque (95:5:0,5 V/V/V).
Le chlorhydrate correspondant est obtenu dans un mélange d'éther-éthanol.
Rendement : 20%
Point de Fusion : 170°C.

**EXEMPLE 29**

**Chlorhydrate de la dioxo-2,4 {[(fluoro-4 benzoyl)-4 perhydroazépinyl-1]-2 éthyl}-3 tétrahydro-1,2,3,4 quinazoline**

Ce composé a été préparé selon le procédé décrit dans l'exemple 10 en utilisant la (fluoro-4 benzoyl)-4 perhydroazépine à la place de la (fluoro-4 phénacyl)-3 pyrrolidine.
Rendement : 60%
Point de Fusion : 238°C.

**EXEMPLE 30**

**Chlorhydrate de la carbamoyl-8 {[(fluoro-4 benzoyl)-4 perhydroazépinyl-1]-2 éthyl}-3 hydroxy-4 méthyl-2 imidazo [1,5a] pyrimidine**

Ce composé a été synthétisé selon le procédé décrit dans l'exemple 9 en utilisant la (fluoro-4 benzoyl)-4 perhydroazépine au lieu de la (fluoro-4 phénacyl)-3 pyrrolidine, au stade A.
Rendement : 25%
Point de Fusion : 〉 260°C

**EXEMPLE 31**

**Chlorhydrate de la {[(fluoro-4 phénacyl)-3 pyrrolidinyl-1]-2 éthyl}-2 oxo-1 phtalazine**

STADE A

{[(tétrahydropyrannyl-2) oxy]-2 éthyl}-2 oxo-1 phtalazine

Additionner à température ambiante une solution de 41,5 g d'hydroxyde de potassium dans 53 ml d'eau à une solution de 61 g de oxo-1 phtalazine dans 430 ml de diméthylsulfoxyde. Après 30 minutes, ajouter à la suspension obtenue 130 g de tétrahydropyrannyl-2 bromo-2 éthyl éther. Laisser agiter 30 heures à température ambiante puis concentrer le tout, reprendre le résidu dans 400 ml d'eau et extraire au dichlorométhane. Concentrer.
Rendement : 80%

Spectre de résonance magnétique nucléaire du proton (400 MHz, solvant $CDCl_3$): 1,3 à 2 ppm,m,6H; 3,3 à 4,8 ppm,m+m 1H+6H; 7,6 à 7,9 ppm,m,3H; 8,15 ppm,s,1H; 8,4 ppm,m,1H

STADE B

(Hydroxy-2 éthyl)-2 oxo-1 2H-phtalazine

Chauffer 15 heures à 55°C sous agitation un mélange de 91 g du produit obtenu dans le Stade A, 500 ml d'acide acétique, 250 ml de tétrahydrofuranne et 150 ml d'eau. Puis concentrer le tout sous 1mm de Hg et purifier l'huile obtenue par chromatographie sur silice 70-230 mesh en utilisant comme éluant un mélange de dichlorométhane-méthanol-ammoniaque (99:1:0,1 V/V/V).
Rendement : 75%

Spectre de résonance magnétique nucléaire du proton (200 MHz, solvant $CDCl_3$): 4,0 ppm,q,2H; 4,9 ppm,t+t,1H+3H; 7,75 ppm,m,3H; 8,2 ppm,s,1H; 8,35 ppm,d,1H

STADE C

(chloro-2 éthyl)-2 oxo-1 2H-phtalazine

Porter 3 heures à reflux un mélange de 45 g du produit obtenu au Stade B, 31 g de chlorure de thionyle et 350 ml de chloroforme. Concentrer le tout, reprendre le résidu dans l'éther éthylique et filtrer le solide obtenu.
Rendement : 70%

Spectre de résonance magnétique nucléaire du proton (200 MHz, solvant $CDCl_3$): 3,95 ppm,t,2H; 4,6 ppm,t,2H; 7,15 à 7,9 ppm,m,3H; 8,2 ppm,s,1H; 8,4 ppm,m,1H

STADE D

Porter 30 heures à reflux un mélange de 8 g du produit obtenu au Stade C, 10 g de (fluoro-4 phénacyl)-3 pyrrolidine, 8,8 g d'hydrogénocarbonate de sodium et 200 ml de méthylbenzène. Puis filtrer et concentrer le tout. L'huile obtenue est purifiée par chromatographie sur silice 70-230 mesh en utilisant comme éluant un mélange de dichlorométhane-méthanol-ammoniaque (98,5:1,5:0,15 V/V/V).

Salifier les 9,3 g de base ainsi obtenue dans l'acétone avec de l'acide chlorhydrique éthanolique.

Rendement : 60%

Point de fusion :182°C

**EXEMPLE 32**

**Chlorhydrate de la dioxo-2,4 {[(fluoro-4 phénacyl)-3 azétidinyl-1]-2 éthyl}-3 tétrahydro-1,2,3,4 quinazoline**

STADE A

(Fluoro-4 phénacyl)-2 cyanoacétate d'éthyle

Additionner à 15°C, une solution de 86,7 g de sodium dans 4500 ml d'éthanol anhydre, à une solution de 528 g de cyanoacétate d'éthyle dans 500 ml d'éthanol anhydre. Après une heure, refroidir à 0°C et additionner à la solution obtenue une solution de 620 g de chloro-2 fluoro-4 acétophénone dans un litre d'éthanol. Laisser le mélange réactionnel 12 heures à température ambiante, puis hydrolyser avec 200 ml d'eau et concentrer. Reprendre le résidu dans un litre d'eau, neutraliser à pH7, extraire cette phase aqueuse au dichlorométhane puis concentrer.

L'huile obtenue est distillée sous 0,06 mmHg.

Rendement : 88%

Point de Fusion : 160-180°C.

Spectre de résonance magnétique nucléaire du proton (200 MHz, solvant DMSO-$d_6$): 1,0-1,3 ppm,t,3H; 3,8 ppm,d,2H; 4-4,3 ppm,q,2H; 4,55 ppm,t,1H; 7,4 ppm,t,2H; 8,1 ppm,dd,2H.

STADE B

[(cyano-2 éthoxycarbonyl)-2 éthyl]-2 (fluoro-4 phényl)-2 dioxolanne-1,3

Chauffer à 95°C un mélange de 906 g de (fluoro-4 phénacyl)-2 cyanoacétate d'éthyle, 2700 ml d'éthylène glycol anhydre, 1300 ml d'orthoformiate d'éthyle, et 20 g d'acide méthanesulfonique. Distiller l'éthanol et le formiate d'éthyle qui se forment. Après 72 heures, jeter le mélange réactionnel sur 10 litres d'une solution à 200 g/l de carbonate de sodium et extraire à l'éther éthylique. Purifier l'huile obtenue par chromatographie sur silice 70-230 mesh en utilisant comme éluant un mélange cyclohexane éther éthylique (85:15 V/V).

Rendement : 75%

Spectre de résonance magnétique nucléaire du proton (200 MHz, solvant $CDCl_3$): 1,2-1,4 ppm,t,3H; 2,4-2,7 ppm,m,2H; 3,7-3,9 ppm,m,3H; 4,0-4,2 ppm,m,2H; 4,2-4,4 ppm,q,2H; 7-7,15 ppm,t,2H; 7,4-7,5 ppm,dd,2H

STADE C

[(aminométhyl-2 éthoxycarbonyl)-2 éthyl]-2 (fluoro-4 phényl)-2 dioxolanne-1,3

Charger un appareil de Parr avec 450 g du produit obtenu au Stade B, 900 ml d'acide acétique, 3000 ml d'éthanol anhydre et 20 g d'oxyde de platine. Hydrogéner sous 5 bars à 45°C. Quand le volume théorique est absorbé, filtrer le catalyseur et évaporer le solvant sous vide. Reprendre le résidu dans 1 litre d'eau, neutraliser à la soude et extraire au dichlorométhane. Purifier l'huile obtenue par chromatographie sur silice 70-230 mesh en utilisant comme éluant un mélange de dichlorométhane, de méthanol et d'ammoniaque (95:5:0,5 V/V/V).

Rendement : 60%

Spectre de résonance magnétique nucléaire du proton (200 MHz, solvant $CDCl_3$): 2 ppm,dd,1H; 2,35 ppm,dd,1H; 2,6 ppm,m,1H; 2,8 ppm,m,2H; 3,7 ppm,m,2H; 4 ppm,m,2H; 4,1 ppm,q,2H; 7 ppm,m,2H; 7,4 ppm,m,2H

STADE D

(fluoro-4 phényl)-2 [(oxo-2 azétidin-3-yl) méthyl]-2 dioxolanne

Additionner à 0°C une solution de 150 g du produit obtenu au Stade C dans 150 ml d'éthoxyétane à une solution de 2 mole d'iodure de méthylmagnésium dans 650 ml d'éthoxyéthane. Après 4 heures à cette température, hydrolyser le mélange réactionnel avec 250 ml d'une solution de chlorure d'ammonium saturée. Décanter et concentrer la phase organique. L'huile obtenue est purifiée par chromatographie sur silice 70-230 mesh. Eluant dichlorométane méthanol ammoniaque (98:2:0,2 V/V/V).
Redement : 72%
Spectre de résonance magnétique nucléaire du proton (200 MHz, solvant $CDCl_3$): 2,1 ppm,dd,1H; 2,5 ppm,dd,1H; 3,05 ppm,m,1H; 3,35 ppm,m,2H; 3,75 ppm,m,2H; 4,0 ppm,m,2H; 5,75 ppm,m,1H; 7,0 ppm,t,2H; 7,4 ppm,dd,2H

STADE E

(fluoro-4 phényl)-2 (azétidin-3-yl méthyl)-2 dioxolanne

A une suspension de 4,6 g d'hydrure de lithium et d'aluminium dans 150 ml d'éthoxy-éthane anhydre, additionner à -20°C une solution de 15 g du produit obtenu au Stade D dans 150 ml de tétrahydrofuranne. Porter ensuite 12 heures à reflux, puis hydrolyser avec 80 ml d'eau et 10 ml de solution d'hydroxyde de sodium à 10%. Purifier l'huile obtenue par chromatographie sur silice 70-230 mesh en utilisant comme éluant un mélange de dichlorométhane, de méthanol et d'ammoniaque (90:10:1 V/V/V).
Rendement 78%
Spectre de résonance magnétique nucléaire du 13C (400 MHz, solvant DMSO-$d_6$): 30,1 ppm; 44,2 ppm; 52,4 ppm; 64,02 ppm; 108,9 ppm; 114,7 ppm; 127,4 ppm; 138,5 ppm; 161,4 ppm

STADE F

Dioxo-2,4 {[[(fluoro-4 phényl)-2 méthyl-2 dioxolanne-1,3]-3 azétidinyl-1]-2 éthyl}-3 tétrahydro-1,2,3,4 quinazoline

Porter 15 heures à reflux un mélange de 11 g du produit obtenu au Stade E, 10,5 g de (chloro-2 éthyl)-3 dioxo-2,4 tétrahydro-1,2,3,4 quinazoline, 6,5 g du carbonate de sodium et 150 ml de toluène. Filtrer, rincer le précipité au dichlorométhane et concentrer. Reprendre le résidu solide obtenu dans un mélange éthoxyéthane-acétate d'éthyle et filtrer le précipité.
Rendement : 70%
Point de Fusion : 185°C
Spectre de résonance magnétique nucléaire du proton (200 MHz, solvant DMSO-$d_6$): 2,0 ppm,d,2H; 2,35 ppm,m,1H; 2,4-2,65 ppm,m+m,2H+2H; 3,3 ppm,m,2H; 3,65 ppm,m,2H; 3,80 ppm,m,2H; 3,95 ppm,m,2H; 7,05-7,25 ppm,dd+td+t,1H+1H+2H; 7,35 ppm,dd,2H; 7,65 ppm,m,1H;; 7,9 ppm,dd,1H

STADE G

Chauffer à reflux pendant 30 minutes un mélange de 10 g du produit obtenu au stade précédent, 150 ml de tétrahydrofuranne et 150 ml d'acide chlorhydrique 2N. Puis concentrer sous vide le tétrahydrofuranne et filtrer pour isoler le chlorhydrate de la dioxo-2,4 {[(fluoro-4 phénacyl)-3 azétidinyl-1]-2 éthyl}-3 tétrahydro-1,2,3,4 quinazoline. Rincer ce précipité à l'acétone et le sécher sous vide.
Rendement : 80%
Point de Fusion : 220°C (décomposition).

**EXEMPLE 33**

**Dichlorhydrate de la {[(fluoro-4 phénacyl)-3 pyrrolidinyl-1]-2 éthyl}-6 méthyl-7 oxo-5 5H-thiazolo [3,2-a] pyrimidine**

Porter au reflux 9,8 g de (fluoro-4 phénacyl)-3 pyrrolidine, 10,31 g de (chloro-2 éthyl)-6 méthyl-7 oxo-5 5H-thiazolo [3,2-a] pyrimidine, 10 g d'hydrogénocarbonate de sodium et 150 ml de toluène pendant 50 heures.

Filtrer et concentrer le toluène sous vide. L'huile obtenue est ensuite purifiée par deux chromatographies sur colonnes de gel de silice 70-230 mesh en utilisant comme éluant, d'abord un mélange de dichlorométhane, de méthanol et d'ammoniaque (93/7/0,7 V/V/V), puis un mélange d'éther, d'hexane et de méthanol (60/30/10 V/V/V).

Le dichlorhydrate correspondant est obtenu dans l'éthanol.
Rendement : 24%
Point de Fusion : 162-164°C

**EXEMPLE 34**

**Chlorhydrate de la (-) dioxo-2,4 {[(fluoro-4 phénacyl)-3 pyrrolidinyl-1]-2 éthyl}-3 tétrahydro-1,2,3,4 quinazoline**

Un mélange de 25,04 g de (fluoro-4 phénacyl)-3 pyrrolidine et de 42 g d'acide (-) di-paratoluoyltartrique dans 600 ml d'éthanol est chauffé 3 minutes à 55°C. L'évaporation du solvant fournit un composé qui après 3 recristallisations dans un mélange d'acétone et de méthanol conduit à 15 g d'un solide blanc.

L'amine est relarguée avec une solution d'hydroxyde de potassium.

Le produit final est ensuite préparé suivant le procédé décrit dans l'exemple 10.
Rendement : 16%
Point de Fusion : 170-171°C
Pureté énantiomérique : 〉 99% (mesurée par HPLC)
Pouvoir rotatoire C = 1% dans $CHCl_3$ :

$$[\alpha]_D^{22} = -22,1$$

**EXEMPLE 35**

**Chlorhydrate de la (+) dioxo-2,4 {[(fluoro-4 phénacyl)-3 pyrrolidinyl-1]-2 éthyl]-3 tétrahydro-1,2,3,4 quinazoline**

Les filtrats des trois recristallisations de l'exemple 34 sont réunis, puis le solvant est évaporé. L'amine est relarguée par une solution d'hydroxyde de potassium. La base ainsi obtenue est salifiée par l'acide (+) di-paratoluoyltartrique dans l'éthanol à 55°C. Le chlorhydrate de la (+) dioxo-2,4 {[(fluoro-4 phénacyl)-3 pyrrolidinyl-1]-2 éthyl}-3 tétrahydro-1,2,3,4 quinazoline est ensuite préparé suivant le procédé décrit dans l'exemple 34.
Rendement : 16%
Point de Fusion : 151°C
Pureté énantiomérique : 〉 99% (mesurée par HPLC)
Pouvoir rotatoire C = 1% dans $CHCl_3$ :

$$[\alpha]_D^{22} = +22,3$$

**TABLEAU I**

**Composés de formule générale I**

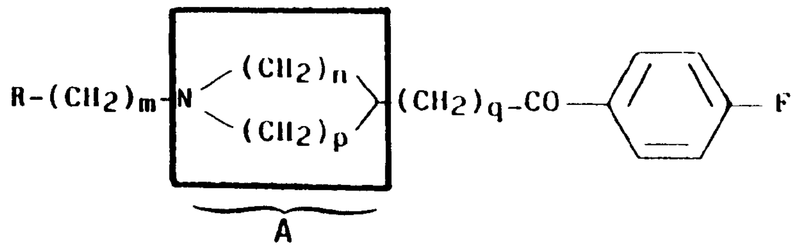

| EXEMPLE N° | R | m | A | q | Spectre RMN (Solvant) |
|---|---|---|---|---|---|
| 1 | 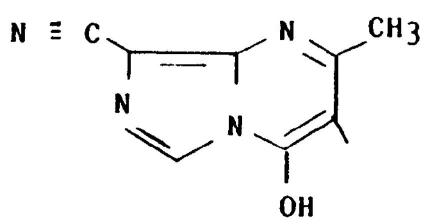 | 2 | 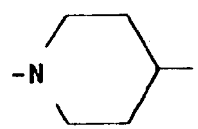 | 0 | RMN-$^1$H (DMSO-$d_6$) Sel 2,0-2,2 ppm,m,4H; 2,35 ppm,s,3H; 2,7-4,0 ppm,m,9H; 7,4-7,7 ppm,m,2H; 8,1 ppm,s,1H; 8-8,4 ppm,m,2H |
| 2 | 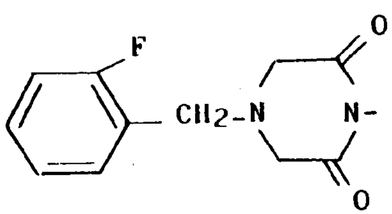 | 3 | 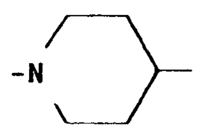 | 0 | RMN-$^1$H (DMSO-$d_6$) Sel 1,8-2,3 ppm,m,6H; 2,8-3,6 ppm,m,9H; 3,6 ppm,s,4H; 3,8 ppm,s,2H; 7,2-7,6 ppm,m,6H; 8,0-8,3 ppm,m,2H; 10,0-11,0 ppm,1H échangeable |

TABLEAU I
(Suite I)

| EXEMPLE N° | R | m | A | q | Spectre RMN (Solvant) |
|---|---|---|---|---|---|
| 3 | 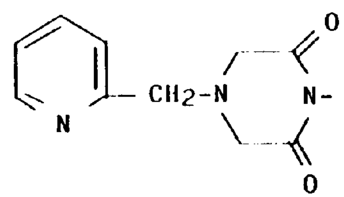 | 3 | 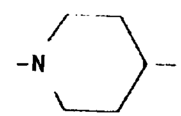 | 0 | RMN-$^{1}$H ($CDCl_3$) Sel 1,8-2,8 ppm,m,6H; 2,8-4,1 ppm,m, 15H; 7,0-7,6 ppm,m,4H; 7,6-8,3 ppm, m,3H; 8,6 ppm,m,1H |
| 4 | 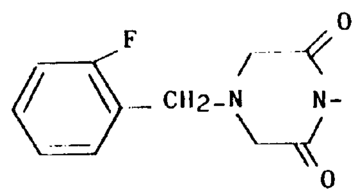 | 4 | 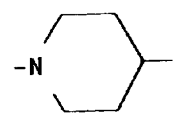 | 0 | RMN-$^{1}$H (DMSO-$d_6$) Sel 1,3-2,2 ppm,m,8H; 2,6-4,0 ppm,s+m+s +m, 2H+2H+4H+7H; 7,1-7,7 ppm,m,6H; 8,0-8,3 ppm,m,2H |
| 5 | 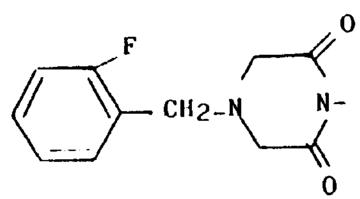 | 3 | 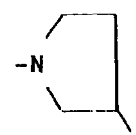 | 1 | RMN-$^{1}$H ($CDCl_3$ + DMSO-$d_6$) Sel 1,2-2,2 ppm,m,4H; 2,5-4,0 ppm,m+m+s+m+m,2H+2H+4H+8H+1H; 4,3 ppm,m, 2H; 4,8-5,5 ppm,1H échangeable; 6,9-7,6 ppm,m,6H; 7,8-8,2 ppm,m,2H 8,0-10 ppm,1H échangeable |

**TABLEAU I**
**(Suite II)**

| EXEMPLE N° | R | m | A | q | Spectre RMN (Solvant) |
|---|---|---|---|---|---|
| 6 | 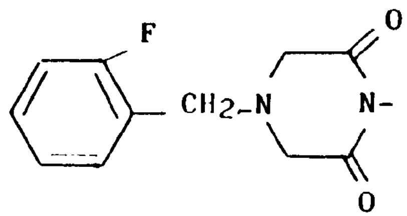 | 3 | 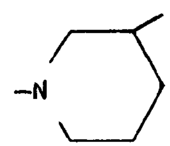 | 1 | RMN-$^1H$ ($CDCl_3$) Sel<br>1,4-3,3 ppm,m+m,6H+9H; 3,4-3,9 ppm, s+m+s,4H+2H+2H; 4,25 ppm,s,4H; 6,0-8,0 ppm,1H échangeable; 7,1-7,8 ppm, m,6H; 7,9-8,3 ppm,m,2H |
| 7 | 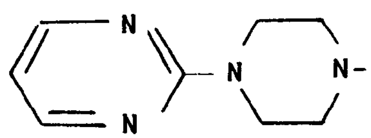 | 3 | 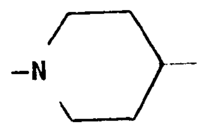 | 0 | RMN-$^1H$ (DMSO-$d_6$) Sel<br>1,6-2,5 ppm,m,6H; 2,7-4,5 ppm,m+m+m,4H+12H+1H; 4,5-5,1 ppm,1H échangeable; 6,85 ppm,t,1H; 7,4 ppm,t,2H; 8-8,3 ppm,dd,2H; 8,5 ppm,d,2H; 10,8-12,2 ppm,1H échangeable |

**TABLEAU I**
**(Suite III)**

| EXEMPLE N° | R | m | A | q | Spectre RMN (Solvant) |
|---|---|---|---|---|---|
| 8 | 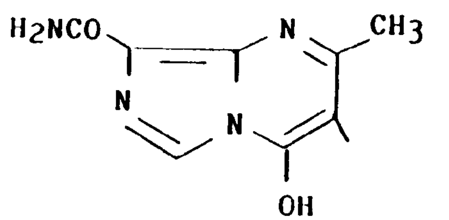 | 2 | 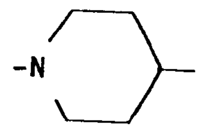 | 0 | RMN-$^1$H (DMSO-$d_6$) Sel 1,9 ppm,m,2H; 2,04 ppm,m,2H; 2,54 ppm,s,3H; 2,93 ppm,m,2H; 3,13 ppm,m,5H; 3,7 ppm,m,2H; 7,23 1H échangeable; 7,4 ppm,t,2H; 7,43 ppm,1H échangeable; 8,1 ppm,dd,2H; 8,14 ppm, s,1H; 9,93 ppm 1H échangeable; 11,6 ppm,m,1H |
| 9 | 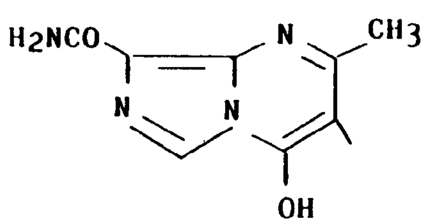 | 2 | 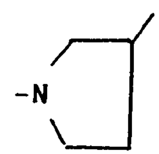 | 1 | RMN-$^1$H (DMSO-$d_6$) Base 1,72 ppm,m,1H; 2,2 ppm,m,1H; 2,53 ppm,s,3H; 2,75 ppm,m,1H; 2,90 ppm,t,2H; 3,07 ppm,m,1H; 3,20 ppm,m,1H; 3,35 ppm,d,5H; 3,62 ppm,m,1H; 3,84 ppm,m,1H; 7,23 ppm,s,1H; 7,35 ppm,dd,2H; 7,41 ppm,s,1H; 8,05 ppm,dd,2H; 8,10 ppm,s,1H; 11,54 ppm,s,1H |

TABLEAU I

(Suite IV)

| FXEMPLE N° | R | m | A | q | Spectre RMN (Solvant) |
|---|---|---|---|---|---|
| 10 | 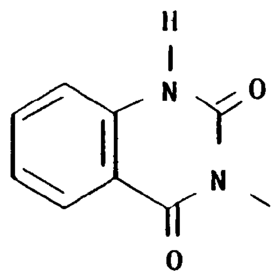 | 2 | 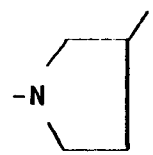 | 1 | RMN-$^1$H (DMSO-$d_6$) Sel<br>1,5-2,6 ppm,m,2H; 1,5-2,6 ppm,m,1H; 2,5-3,0 ppm,m,2H; 3,0-3,7 ppm,m,6H; 4,2 ppm,t,2H; 7,0-8,3 ppm,m,8H |
| 11 | 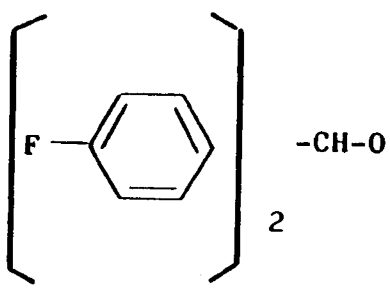 | 2 | 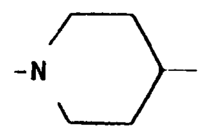 | 0 | RMN-$^1$H ($CDCl_3$) Base<br>1,9 ppm,m,4H; 2,8 ppm,m,4H; 2,7 ppm,t,2H; 3,2 ppm,m,1H; 3,6 ppm,t,2H; 5,4 ppm,s,1H; 7,2 ppm,m,10H; 8,0 ppm, m,2H |

TABLEAU I
(Suite V)

| EXEMPLE N° | R | m | A | q | Spectre RMN (Solvant) |
|---|---|---|---|---|---|
| 12 | 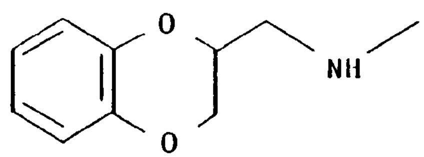 | 3 | 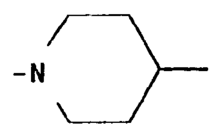 | 0 | RMN-$^1$H ($CDCl_3$) Base<br>1,0-3,7 ppm,m+m+m,6H+1H+10H;2,4-3,1 ppm,1H échangeable; 4,0-4,5 ppm,m, 3H; 6,9 ppm,m,4H; 7,0 -7,4 ppm,m,2H; 7,2-8,9 ppm,m,2H |
| 13 | 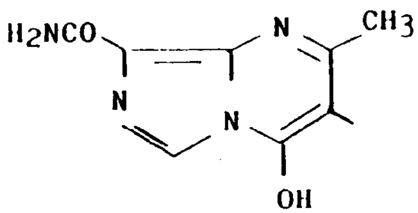 | 2 | 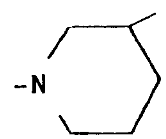 | 1 | RMN-$^{13}$C (DMSO-d$_6$) Base<br>17,4 ppm; 19,5 ppm; 30,0 ppm; 54,6 ppm; 55,6 ppm; 100,2 ppm; 115,2 ppm; 115,3 ppm; 123,3 ppm; 130,8 ppm; 132,6 ppm; 133,4 ppm; 151,3 ppm; 155,8 ppm; 165,0 ppm; 166,3 ppm; 196,6 ppm; |

**TABLEAU I**
**(Suite VI)**

| EXEMPLE N° | R | m | A | q | Spectre RMN (Solvant) |
|---|---|---|---|---|---|
| 14 | 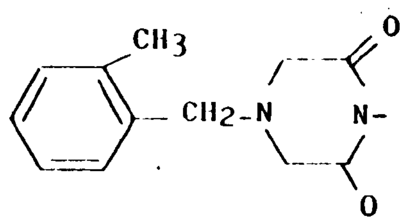 | 3 | 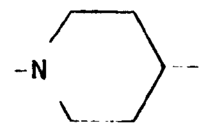 | 0 | RMN-$^1H$ (DMSO-$d_6$) Sel 1,5-2,5 ppm,s+m,3H+7H; 2,8-4,0 ppm, m+s+s+m,2H+2H+4H+6H; 7,0-7,6 ppm,m, 6H; 8,0-8,4 ppm,m, 2H; 10-11,5 ppm, 1H échangeable |
| 15 | 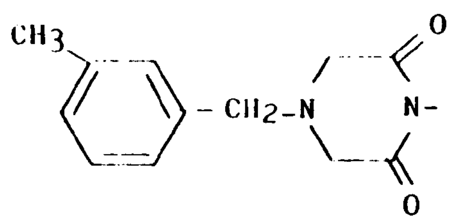 | 3 | 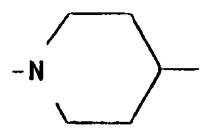 | 0 | RMN-$^1H$ (DMSO-$d_6$) Sel 1,6-2,4 ppm,m,7H; 2,3 ppm,s, 3H; 2,4-3,9 ppm,m+s+s+m,2H+2H+4H+6H; 7,0-7,7 ppm,m,6H; 8,0-8,4 ppm,m,2H; 9,5-11,0 ppm,1H échangeable |
| 16 | 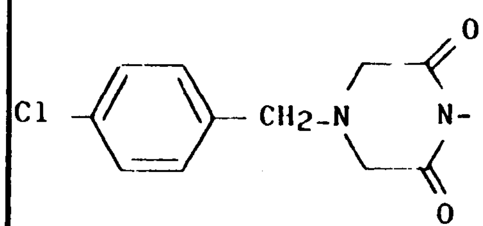 | 3 | 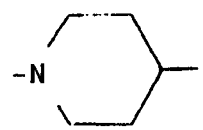 | 0 | RMN-$^1H$ ($CDCl_3$) Sel 1,6-2,5 ppm,m,7H; 2,7-4,0 ppm,m+s+s+m,2H+2H+4H+6H; 7,0-7,6 ppm,m,6H; 7,8-8,3 ppm,m,2H; 10,0-11,0 ppm,m,1H échangeable |

TABLEAU I
(Suite VII)

| EXEMPLE N° | R | m | A | q | Spectre RMN (Solvant) |
|---|---|---|---|---|---|
| 17 | 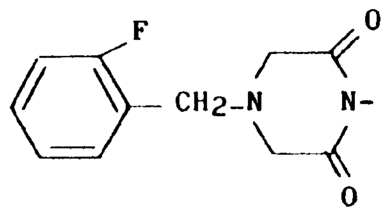 | 2 | 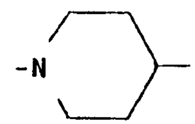 | 0 | RMN-$^1$H ($CDCl_3$) Sel 1,8-2,2 ppm,m,4H; 2,8-4,0 ppm,m,9H; 3,3 ppm,m,4H; 3,6 ppm,s,2H; 7,7-7,1 ppm,m,6H; 8,0-8,3 ppm,m,2H; 10,0-11,0 ppm, 1H échangeable |
| 18 | 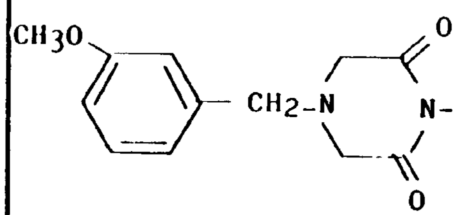 | 3 | 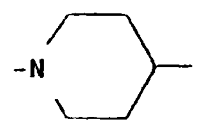 | 0 | RMN-$^1$H (DMSO-$d_6$) Sel 1,8-2,8 ppm,m,7H; 2,8-3,6 ppm, m,6H; 3,5 ppm,s,4H; 3,7 ppm,s,2H; 3,8-4,0 ppm,m,2H; 3,9 ppm,s,3H; 6,8-7,5 ppm, m,6H; 8,0-8,3 ppm,m,2H |
| 19 | 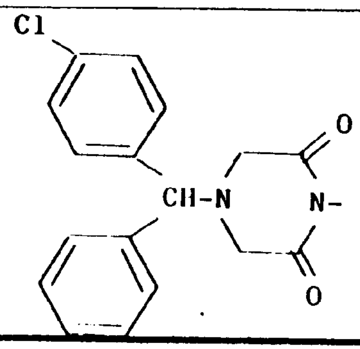 | 2 | 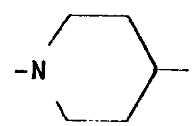 | 0 | RMN-$^1$H (DMSO-$d_6$) Sel 1,7-2,3 ppm,m,4H; 2,7-4,0 ppm,m,7H; 3,5 ppm,s,4H; 3,9-4,2 ppm,m,2H; 4,8 ppm,s,1H; 7,2-7,6 ppm,m,11H; 8,0-8,4 ppm,m,2H; 10,5-11,5 ppm,1H échangeable |

**TABLEAU I**
**(Suite VIII)**

| EXEMPLE N° | R | m | A | q | Spectre RMN (Solvant) |
|---|---|---|---|---|---|
| 20 | 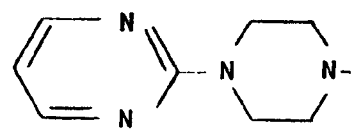 | 3 | 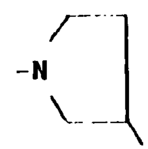 | 1 | RMN-$^1$H (DMSO-d$_6$) Sel 1,5-4,2 ppm,m+m+m,4H+14H+5H; 4,5-5,0 ppm,1H échangeable; 6,7-6,9 ppm,t, 1H; 7,2-7,5 ppm,t,2H; 7,9-8,2 ppm, dd,2H; 8,4-8,5 ppm,d,2H; 11,0-12,0 ppm 1H échangeable |
| 21 | 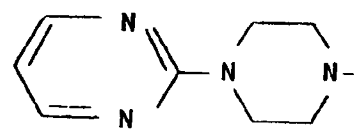 | 2 | 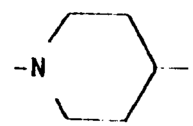 | 0 | RMN-$^1$H (D$_2$O) Sel 1,8-2,5 ppm,m,4H; 3,2-4,3 ppm,m, 17H; 6,9 ppm,t,1H; 7,1-7,6 ppm,m,2H; 7,8-8,3 ppm,m,2H; 8,5 ppm,d,2H |
| 22 | 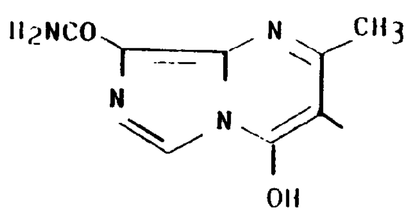 | 3 | 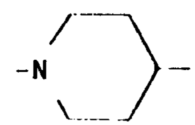 | 0 | RMN-$^{13}$C (DMSO-d$_6$) Sel 21,6 ppm; 22,7 ppm; 25,6 ppm; 50,9 ppm; 55,5 ppm; 103,5 ppm; 114,9 ppm; 115,7 ppm; 123,3 ppm; 131,1 ppm; 132,0 ppm; 132,8 ppm; 150,2 ppm; 156,1 ppm; 164,4 ppm; 165,0 ppm; 199,5 ppm |

**TABLEAU I**
**(Suite IX)**

| EXEMPLE N° | R | m | A | q | Spectre RMN (Solvant) |
|---|---|---|---|---|---|
| 23 | 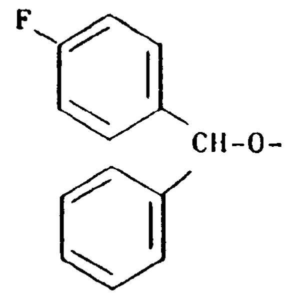 | 2 | 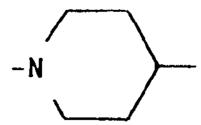 | 0 | RMN-$^1$H (DMSO-$d_6$) Sel<br>1,7-2,3 ppm,m,4H; 2,7-4,0 ppm,m+m+m+m,2H+4H+2H+1H; 5,7 ppm,s,1H; 6,1 ppm,s,2H; 6,9-7,6 ppm,m,11H; 7,85-8,25 ppm,m,2H |
| 24 | 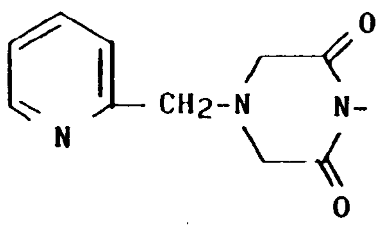 | 2 | 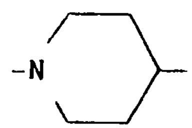 | 0 | RMN-$^1$H (DMSO-$d_6$) Sel<br>1,8-2,3 ppm,m,4H; 3,0-3,7 ppm,m,7H; 3,9 ppm,s,4H; 4,0-4,2 ppm,m,2H; 4,4 ppm,s,2H; 7,2-7,6 ppm,t,2H; 7,8-8,6 ppm,m,5H; 8,6-9,0 ppm,m,1H |

**TABLEAU I**
**(Suite X)**

| EXEMPLE N° | R | m | A | q | Spectre RMN (Solvant) |
|---|---|---|---|---|---|
| 25 | 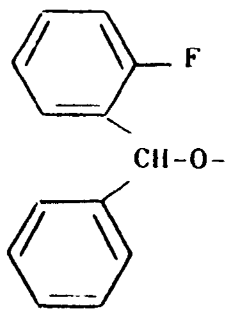 | 2 | 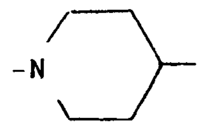 | 0 | RMN-$^1$H ($CDCl_3$) Sel 2,0-2,5 ppm,m,4H; 3,0-4,0 ppm,m,9H; 5,25 ppm,s,1H; 6,3 ppm,s,2H; 7,1-7,6 ppm,m,10H; 7,9-8,2 ppm,dd,2H; 12-13 ppm,1H échangeable |
| 26 | 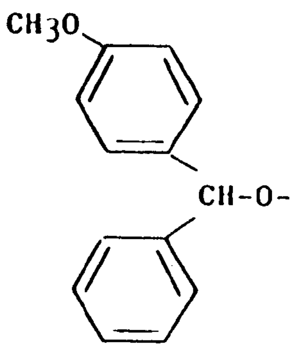 | 2 | 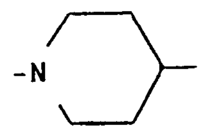 | 0 | RMN-$^1$H (DMSO-$d_6$) Sel 1,5-2,3 ppm,m,4H; 2,7-4,0 ppm,m,9H; 3,75 ppm,s,3H; 5,5 ppm,s,1H; 6,1 ppm,s,2H; 6,8-7,6 ppm,m,11H; 8,0-8,3 ppm,m,2H |

TABLEAU I
(Suite XI)

| EXEMPLE N° | R | m | A | q | Spectre RMN (Solvant) |
|---|---|---|---|---|---|
| 27 | 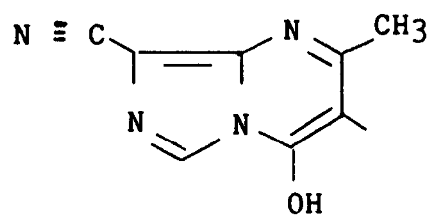 | 2 | 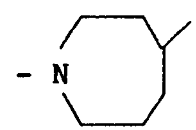 | 0 | RMN-13C (DMSO) Sel 17,2 ppm; 19,9 ppm; 22,2 ppm; 25,3 ppm; 29,0 ppm; 43,3 ppm; 51,7 ppm; 53,8 ppm; 54,6 ppm; 92,1 ppm; 101,2 ppm; 114,4 ppm; 115,4 ppm; 115,7 ppm; 126,0 ppm; 131,0 ppm; 131,2 ppm; 132,4 ppm; 137,6 ppm; 151,1 ppm; 155,5 ppm; 163,3 ppm; 166,7 ppm; 200,7 ppm |

TABLEAU I
(Suite XII)

| EXEMPLE N° | R | m | A | q | Spectre RMN (Solvant) |
|---|---|---|---|---|---|
| 28 | 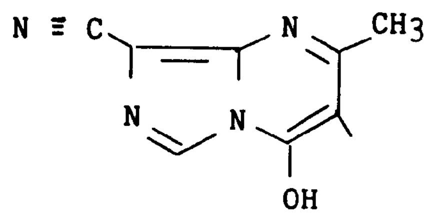 | 2 | 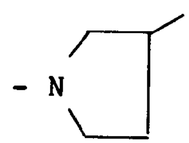 | 1 | RMN-$^{13}C$ (DMSO-d6) Sel 21,0 ppm; 29,0 ppm; 31,8 ppm; 41,7 ppm; 52,1 ppm; 53,0 ppm; 57,5 ppm; 91,6 ppm; 101 ppm; 114,7 ppm; 115,7 ppm; 126,5 ppm; 130,8 ppm; 133,0 ppm; 137,6 ppm; 150,9 ppm; 155,5 ppm; 165 ppm; 197,2 ppm |
| 29 | 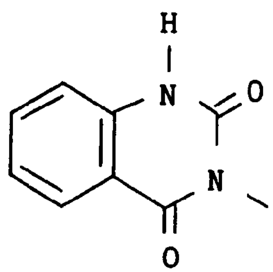 | 2 | 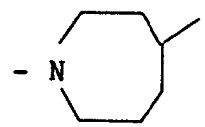 | 0 | RMN-$^{1}H$ (DMSO-d6) Sel 1,4-2,2 ppm,m+m+m,1+1+4H; 3,0-4,1 ppm,m+m+m,1+1+5H; 4,3 ppm,m,2H; 7,2 ppm,m,2H; 7,35 ppm,t,2H; 7,7 ppm,t, 1H; 7,95 ppm,d,1H; 8,1 ppm,dd,2H |

**TABLEAU I**
**(Suite XIII)**

| EXEMPLE N° | R | m | A | q | Spectre RMN (Solvant) |
|---|---|---|---|---|---|
| 30 | 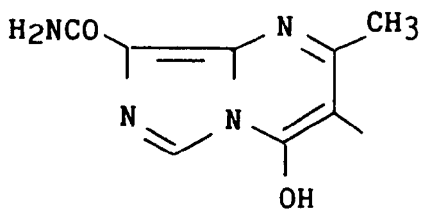 | 2 | 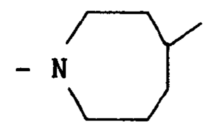 | 0 | RMN-$^{13}C$ (DMSO-$d_6$) Sel 17,2 ppm; 19,8 ppm; 42,7 ppm; 43,6 ppm; 54,3 ppm; 99,9 ppm; 115,0 ppm; 115,6 ppm; 115,9 ppm; 123,4 ppm; 131,2 ppm; 131,3 ppm; 131,9 ppm; 132,5 ppm; 151,4 ppm; 155,8 ppm; 163,3 ppm; 164,3 ppm; 166,6 ppm; 200,6 ppm; 200,9 ppm |
| 31 | 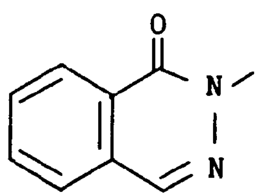 | 2 | 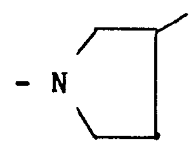 | 1 | RMN-$^{1}H$ (DMSO-$d_6$) Sel 1,7-2,2 ppm,m,2H; 2,8 ppm,m,1H; 3,0 ppm,m,2H; 3,2-3,9 ppm,m,6H; 4,5 ppm,t,2H; 7,3-8,5 ppm,m,9H |

TABLEAU I
(Suite XIV)

| EXEMPLE N° | R | m | A | q | Spectre RMN (Solvant) |
|---|---|---|---|---|---|
| 32 | 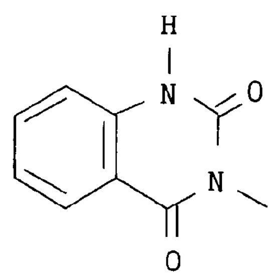 | 2 | 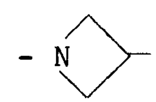 | 1 | RMN-$^1$H (DMSO-$d_6$) Sel<br>3-3,3 ppm,m,1H; 3,4-3,7 ppm,m+d,2H +2H; 4,1 ppm,m,2H; 3,9 ppm,m,2H; 4,25 ppm,m,2H; 7,15-7,30 ppm,d+t,1H+1H; 7,4 ppm,t,2H; 7,7 ppm,t,1H; 7,95 ppm,d,1H; 8,05 ppm,dd,2H |
| 33 | 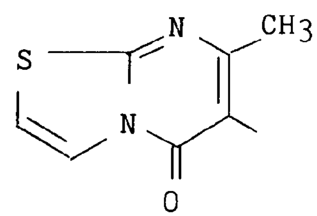 | 2 | 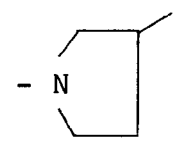 | 1 | RMN-$^1$H (DMSO-$d_6$) Sel<br>1,6-2,0 ppm,m,1H; 2,1-2,4 ppm,m,1H; 2,45 ppm,s,3H; 2,5-4,2 ppm,m,11H; 7,26-7,6 ppm,d+m,1H+2H; 7,95-8,15 ppm,m,3H; 11-11,5 ppm,m,2H échangeables |

## ETUDE PHARMACOLOGIQUE

### EXEMPLE 36

### Antagonisme à l'histamine

Des cobayes albinos mâles (350-400 g), soumis à une diète hydrique pendant 18 heures avant l'essai, sont anesthésiés au carbamate d'éthyle à la dose de 1,25 g/kg par voie intrapéritonéale. Un catheter est introduit dans une artère carotide pour mesurer la pression artérielle au moyen d'une cellule de pression P231D reliée à un enregistreur Gould 2400 ®. Un autre catheter est introduit dans une veine jugulaire et sert à injecter les composés à tester. La trachée est canulée et le cobaye est mis en respiration assistée à l'aide d'un respirateur Havard pour petits animaux.

La température du cobaye est maintenue aux environs de 37°C à l'aide d'une lampe chauffante. Une aiguille piquée dans la canule trachéale est reliée à une cellule de pression P50 permettant d'enregistrer la pression trachéale.

Les cobayes sont prétraités par la d-Tubocurarine (1 mg/kg i.v.). On injecte ensuite l'histamine à la dose de 10 µg/kg par voie veineuse. Cette dose provoque une bronchoconstriction et entraîne une augmentation de la pression trachéale. Les injections d'histamine sont répétées plusieurs fois à 10 minutes d'intervalle jusqu'à stabilisation de la réponse. Les composés de l'invention sont ensuite injectés par voie i.v. à doses cumulatives et la dose inhibant de 100% l'augmentation de la pression trachéale causée par l'injection de l'histamine ($ID_{100}$) est recherchée. l'$ID_{100}$ des composés de l'invention est entre 10 et 250 µg/kg.

### EXEMPLE 37

### Antagonisme 5-$HT_2$

Des rats Sprague-Dawley mâles (350-400 g) sont anesthésiés au pentobarbital par voie i.p. (45 mg/kg). La trachée est canulée et les animaux sont placés en respiration artificielle. Les nerfs vagues sont sectionnés. Un catheter est placé dans une artère carotide pour enregistrer la pression artérielle. Un second cathéter est placé dans la veine du pénis et sert aux injections. On prend la température des animaux et on la maintient à 37°C. Les rats sont prétraités par la d-Tubocurarine (1 mg/kg i.v.) et la prazosine (1 mg/kg i.v.). La 5-hydroxytryptamine est injectée à la dose de 100 µg/kg i.v. deux fois à 10 minutes d'intervalle de façon à déterminer l'élévation de la pression artériellle systolique de chaque rat. 10 minutes après, on injecte les composés de l'invention à la dose la plus faible et l'injection de la 5-hydroxytryptamine est refaite 10 minutes après. 3 ou 4 doses cumulatives des composés de l'invention sont testées de la même manière. On calcule les pourcentages de la réponse hypertensive, obtenus aux différentes doses afin de déterminer l'$ID_{50}$, dose inhibant de 50% la réponse hypertensive. Les résultats de cette étude sont indiqués dans le Tableau II.

TABLEAU II

| COMPOSE | ID50 (µg/kg i.v.) |
|---|---|
| EXEMPLE 1 | 4,5 |
| EXEMPLE 2 | 14,0 |
| EXEMPLE 6 | 3,7 |
| EXEMPLE 8 | 18,0 |
| EXEMPLE 9 | 10,0 |
| EXEMPLE 10 | 5,4 |

**EXEMPLE 38**

**Antagonisme $\alpha_1$**

Des rats Sprague-Dawley de sexe mâle (300-400 g) ayant subi une diète hydrique, sont anesthésiés à l'éther éthylique. Une canule est placée dans la trachée. La moëlle épinière est détruite au moyen d'une tige en acier et la respiration artificielle immédiatement commencée. Les nerfs vagues sont sectionnés. Les artères carotides sont ligaturées, un catheter est placé dans l'une d'elles pour enregistrer la pression artérielle. Un second catheter est placé dans la veine du pénis et sert aux injections. On prend la température des animaux et on la maintient à 36°C. Les rats sont prétraités par un β-bloquant (Tertatolol 100 µg/kg i.v.). La phénylé phrine est injectée à la dose de 4 µg/kg i.v.. Deux injections identiques sont faites à 10 minutes d'intervalle. Les composés de l'invention et la kétansérine sont injectés à la dose la plus faible et l'injection de phényléphrine est refaite 10 minutes après. 3 ou 4 doses cumulatives des composés de l'invention et de la kétansérine sont testées de la même manière. On calcule les pourcentages d'inhibition de la réponse hypertensive, obtenus aux différentes doses afin de déterminer l'$ID_{50}$.

Les résultats de cette étude sont donnés dans le Tableau III. Comme les résultats de ce Tableau le démontrent, les composés de l'invention sont des $\alpha_1$-antagonistes beaucoup plus puissants que la kétansérine.

TABLEAU III

| COMPOSE | ID50 (µg/kg i.v.) |
|---|---|
| EXEMPLE 2 | 376 |
| EXEMPLE 4 | 369 |
| EXEMPLE 5 | 494 |
| EXEMPLE 8 | 240 |
| EXEMPLE 9 | 26 |
| EXEMPLE 10 | 18 |
| EXEMPLE 13 | 147 |
| KETANSERINE | 3.600 |

**EXEMPLE 39**

**Antagonisme 5-HTP**

On utilise 4 rats Wistar femelles (270±30 g) étant à jeun depuis environ 24 heures. Au début de l'essai on leur administre la solution "contrôle" ou les solutions de la 5-hydroxytryptophane à la dose de 320 mg/kg. Les animaux sont ensuite mis en observation dans des cages transparentes. Lors de l'étude trois paramètres sont évalués : Le "forepaw treading", le "flat body posture" et le "head-twiches". Le "forepaw treading" correspond à un pédalage des membres antérieurs. Ce paramètre est mesuré 80 minutes après l'administration de la l,5-hydroxytryptophane et pendant une période d'observation de dix minutes. Ce temps est divisé en périodes de 5 secondes et si un mouvement est observé sous cette période le score de 1 est noté, le maximum de score étant de 30 pour les dix minutes d'observation et pour chaque animal. Le paramètre "head-twiches" est relatif au nombre de secousses de la tête des animaux observées pendant 10 minutes. Ce paramètre est évalué 90 minutes après l'administration de la 1,5-hydroxytryptophane et pendant une période de 10 minutes. Le "flat-body posture" correspond à un aplatissement du corps qui dure plus de 10 minutes. Ce paramètre est évalué pendant tout le temps d'observation des animaux.

Les résultats de ces études qui sont indiqués dans les Tableaux IV et V démontrent que les composés de l'invention sont des antagonistes puissants de 5-HTP. Les résulats présentés dans le Tableau V démontrent aussi que les composés de l'invention sont bien absorbés par voie orale, ce qui constitue un avantage très

important en thérapeutique.

TABLEAU IV

Antagonisme 5-HTP Composés testés par voie subcutanée

| COMPOSE | DE50 (mg/kg) | | |
|---|---|---|---|
| | FPT * | FBP ** | HT *** |
| EXEMPLE 1 | > 40 | 0,04 | > 40 |
| EXEMPLE 2 | 10 | 0,31 | 5 |
| EXEMPLE 3 | 1,25 | 0,31 | 5 |
| EXEMPLE 4 | 5 | 10 | 10 |
| EXEMPLE 5 | 10 | 0,31 | > 10 |
| EXEMPLE 6 | 2,5 | 1,25 | 1,25 |
| EXEMPLE 7 | 1,25 | 1,25 | > 10 |
| EXEMPLE 10 | 0,31 | 0,16 | 2,5 |
| EXEMPLE 15 | 10 | 2,5 | 5 |
| EXEMPLE 18 | 5 | 2,5 | 5 |
| EXEMPLE 21 | 2,5 | 0,63 | 1,25 |

TABLEAU V

| COMPOSE | DE50 (mg/kg) | | |
|---|---|---|---|
| | FPT * | FBP ** | HT *** |
| EXEMPLE 8 | 10 | 0,63 | > 10 |

FPT * = forepaw treading

FBP ** = flat body posture

HT *** = head-twiches

**EXEMPLE 40**

**Comprimés dosés à 10 mg de chlorhydrate de la dioxo-2,4 {[(fluoro-4 phénacyl)-3 pyrrolidinyl-1]-2 éthyl} tétrahydro-1,2,3,4 quinazoline (DFPPETQ)**

| | |
|---|---|
| DFPPETQ .......................... | 10 g |
| Amidon de blé .................... | 100 g |
| Amidon de maïs ................... | 20 g |
| Stéarate de magnésium ............ | 15 g |
| Talc ............................. | 20 g |

pour 1000 comprimés à 10 mg de principe actif.

## Revendications

**Revendications pour les Etats contractants suivants: AT, BE, CH, DE, DK, FR, GB, IT, LI, LU, NL, SE**

**1.** Composés de formule générale I:

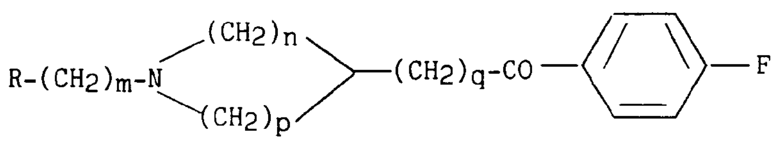

(I)

dans laquelle:
- m représente un nombre entier de 2 à 4,
- n et p identiques ou différents représentent chacun un nombre entier de 1 à 3, n et p ne représentant toutefois pas simultanément le nombre 3,
- q représente 0 ou 1

R représente :

. ou bien un groupement de formule (A):

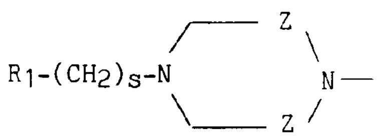

(A)

dans laquelle s représente un nombre entier de 0 à 4, Z représente un radical méthylène ou un radical carbonyle et $R_1$ représente soit un radical phényle (éventuellement substitué par un ou plusieurs atomes d'halogène, par un radical alcoyle inférieur linéaire ou ramifié contenant de 1 à 5 atomes de carbone ou un radical alcoxy inférieur contenant de 1 à 5 atomes de carbone) soit un radical diphénylméthylène (éventuellement substitué par un ou plusieurs atomes d'halogène, par un radical alcoyle

inférieur ou un radical alcoxy inférieur), soit un cycle insaturé de cinq ou six chaînons comportant un ou deux atomes d'azote,

. ou bien un radical de formule (B)

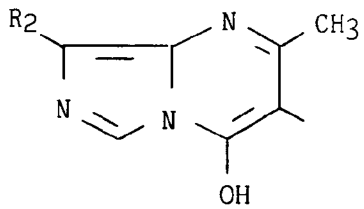

(B)

dans laquelle $R_2$ représente un radical carbamoyle, un radical cyano, un radical carboxy ou un radical alcoxycarbonyle contenant de 2 à 7 atomes de carbone,

. ou bien un radical dioxo-2,4 tétrahydro-1,2,3,4 quinazolinyle à la condition toutefois que, dans ce cas, n et p ne représentent pas simultanément le nombre 2,

. ou bien un radical oxo-1 phtalazinyle, à la condition que n et p ne représentent pas simultanément le nombre 2,

. ou bien un radical oxo-5 thiazolo [3,2-a] pyrimidinyle (éventuellement substitué par un ou plusieurs atomes d'halogène, par un radical alcoyle linéaire ou ramifié contenant de 1 à 5 atomes de carbone ou un radical alcoxy inférieur, contenant de 1 à 5 atomes de carbone), à la condition que n et p ne représentent pas simultanément le nombre 2,

. ou bien un groupement benzhydryloxy (dont les radicaux phényles sont éventuellement substitués par un ou plusieurs atomes d'halogène, par un radical alcoyle linéaire ou ramifié contenant de 1 à 5 atomes de carbone ou un radical alcoxy contenant de 1 à 5 atomes de carbone),

. ou bien un groupement de formule C :

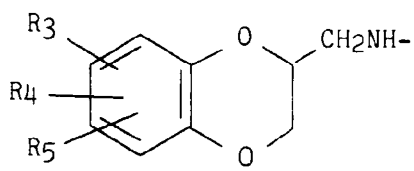

(C)

dans lequel $R_3$, $R_4$, $R_5$ identiques ou différents représentent chacun un atome d'halogène, un radical alcoxy de 1 à 5 atomes de carbone ou un radical alcoyle linéaire ou ramifié de 1 à 5 atomes de carbone,

leurs stéréoisomères possibles, et leurs sels d'addition à un acide minéral ou organique, pharmaceutiquement acceptable.

2. La cyano-8 {[(fluoro-4 benzoyl)-4 pipéridino]-2 éthyl}-3 hydroxy-4 méthyl-2 imidazo [1,5-a] pyrimidine, composé répondant à la formule générale I selon la revendication 1 et ses sels d'addition à un acide minéral ou organique pharmaceutiquement acceptable.

3. La dioxo-2,4 {[(fluoro-4 phénacyl)-3 pyrrolidinyl-1]-2 éthyl}-3 tétrahydro-1,2,3,4 quinazoline, composé répondant à la formule générale I selon la revendication 1, ses isomères optiques et ses sels d'addition à un acide minéral ou organique pharmaceutiquement acceptable.

4. La carbamoyl-8 {[(fluoro-4 phénacyl)-3 pyrrolidinyl-1]-2 éthyl}-3 hydroxy-4 méthyl-2 imidazo [1,5-a] pyrimidine, composé répondant à la formule générale I selon la revendication 1 et ses sels d'addition à un acide minéral ou organique pharmaceutiquement acceptable.

5. La {[(fluoro-4 benzoyl)-4 pipéridino]-3 propyl}-1 (fluoro-2 benzyl)-4 pipérazine dione-2,6, composé répondant à la formule générale I selon la revendication 1 et ses sels d'addition à un acide minéral ou organique pharmaceutiquement acceptable.

6. La {[(fluoro-4 benzoy)-4 pipéridino]-3 propyl}-1 (pyrimidinyl-2)-4 pipérazine, composé répondant à la formule générale I selon la revendication 1 et ses sels d'addition à un acide minéral ou organique pharmaceutiquement acceptable.

7. Procédé de préparation des composés de la formule générale I, selon la revendication 1, caractérisé en ce que:
soit
l'on condense
ou bien
un composé de formule générale II:

$$R\text{-}(CH_2)_m\text{-}X \qquad \textbf{(II)}$$

dans laquelle R et m ont la même signification que pour la formule I selon la revendication 1 et X représente un groupe partant tel qu'un atome d'halogène, un radical mésyle ou un radical tosyle,
avec une amine de formule générale III

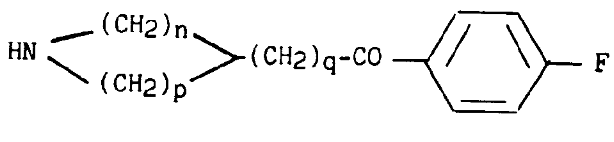
(III)

dans laquelle n, p, et q ont la signification indiquée pour la formule I, selon la revendication 1,
ou bien
que l'on condense un composé de formule générale IV:

**RH (IV)**

dans laquelle R a la même signification que pour la formule I, selon la revendication 1,
avec un composé de formule V:

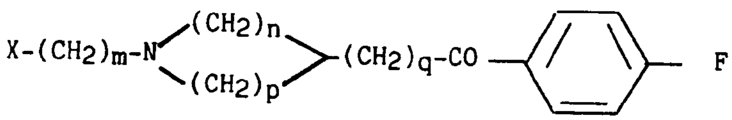
(V)

dans laquelle la signification de m, n, p, et q est identique à celle donnée pour la formule I selon la revendication 1 et X représente un groupe partant dont la définition est identique à celle donnée pour la formule générale II,
pour former les composés de formule I, selon la revendication 1,
soit:
l'on cyclise un dérivé de l'amino-4 imidazole avec un composé de formule VI:

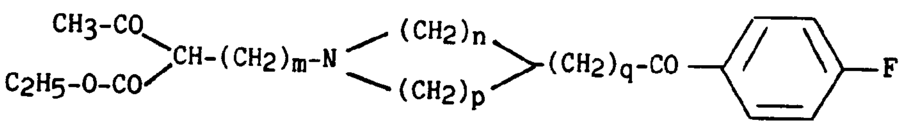
(VI)

dans laquelle m, n, p, et q ont la signification donnée pour la formule I, selon la revendication 1,
pour former les composés de la formule I, selon la revendication 1, dans laquelle R représente un radical de formule B et m, n, p et q ont la signification indiquée pour la formule I,
lesquels ensuite
si l'on désire, sont séparés en leurs stéréoisomères possibles ou/et salifiés par un acide organique ou minéral, pharmaceutiquement acceptable, pour former les sels d'addition correspondants.

8. Composition pharmaceutique contenant comme principe actif un composé selon l'une quelconque des revendications 1 à 6, en association ou en mélange avec un excipient ou un véhicule inerte non toxique, pharmaceutiquement acceptable.

9. Composition pharmaceutique selon la revendication 8, renfermant le principe actif à la dose de 0,5 à 100 mg.

10. Composition pharmaceutique selon les revendications 8 et 9 renfermant comme principe actif au moins un composé selon les revendications 1 à 6 utilisable dans le traitement des maladies nécessitant des antagonistes adrénergiques-$\alpha_1$, de la sérotonine et de l'histamine.

**Revendications pour l'Etat contractant suivant : ES**

1. Procédé de préparation des composés de formule générale I:

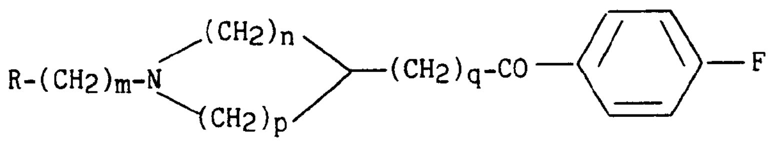

(I)

dans laquelle:

- m représente un nombre entier de 2 à 4,
- n et p identiques ou différents représentent chacun un nombre entier de 1 à 3, n et p ne représentant toutefois pas simultanément le nombre 3,
- q représente 0 ou 1

R représente :

. ou bien un groupement de formule (A):

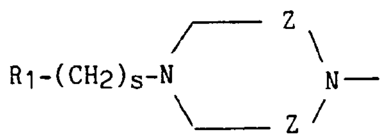

(A)

dans laquelle s représente un nombre entier de 0 à 4, Z représente un radical méthylène ou un radical carbonyle et $R_1$ représente soit un radical phényle (éventuellement substitué par un ou plusieurs atomes d'halogène, par un radical alcoyle inférieur linéaire ou ramifié contenant de 1 à 5 atomes de carbone ou un radical alcoxy inférieur contenant de 1 à 5 atomes de carbone) soit un radical diphénylméthylène (éventuellement substitué par un ou plusieurs atomes d'halogène, par un radical alcoyle inférieur ou un radical alcoxy inférieur), soit un cycle insaturé de cinq ou six chaînons comportant un ou deux atomes d'azote,

. ou bien un radical de formule (B) :

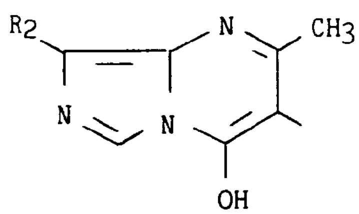

(B)

dans laquelle $R_2$ représente un radical carbamoyle, un radical cyano, un radical carboxy ou un radical alcoxycarbonyle contenant de 2 à 7 atomes de carbone,

. ou bien un radical dioxo-2,4 tétrahydro-1,2,3,4 quinazolinyle à la condition toutefois que, dans ce cas, n et p ne représentent pas simultanément le nombre 2,

. ou bien un radical oxo-1 phtalazinyle, à la condition que n et p ne représentent pas simultanément le nombre 2,

. ou bien un radical oxo-5 thiazolo [3,2-a] pyrimidinyle (éventuellement substitué par un ou plusieurs atomes d'halogène, par un radical alcoyle linéaire ou ramifié contenant de 1 à 5 atomes de carbone ou un radical alcoxy inférieur, contenant de 1 à 5 atomes de carbone), à la condition que n et p ne représentent pas simultanément le nombre 2,

. ou bien un groupement benzhydryloxy (dont les radicaux phényles sont éventuellement substitués par un ou plusieurs atomes d'halogène, par un radical alcoyle linéaire ou ramifié contenant de 1 à 5 atomes de carbone ou un radical alcoxy contenant de 1 à 5 atomes de carbone),

. ou bien un groupement de formule C :

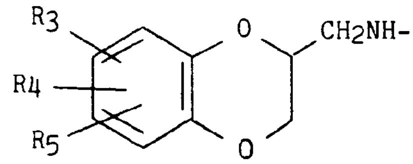

(C)

dans lequel $R_3$, $R_4$, $R_5$ identiques ou différents représentent chacun un atome d'halogène, un radical alcoxy de 1 à 5 atomes de carbone ou un radical alcoyle linéaire ou ramifié de 1 à 5 atomes de carbone,

leurs stéréoisomères possibles, et leurs sels d'addition à un acide minéral ou organique, pharmaceutiquement acceptable,
caractérisé en ce que :
soit
l'on condense
ou bien
un composé de formule générale II :

$$R\text{-}(CH_2)_m\text{-}X \qquad (II)$$

dans laquelle R et m ont la même signification que pour la formule I selon la revendication 1 et X représente un groupe partant tel qu'un atome d'halogène, un radical mésyle ou un radical tosyle,
avec une amine de formule générale III

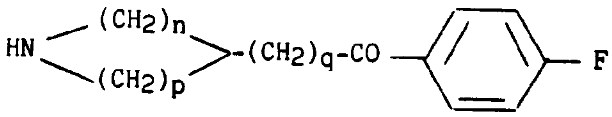

(III)

dans laquelle n, p, et q ont la signification indiquée pour la formule I, selon la revendication 1,
ou bien
que l'on condense un composé de formule générale IV:

**RH (IV)**

dans laquelle R a la même signification que pour la formule I, selon la revendication 1,
avec un composé de formule V:

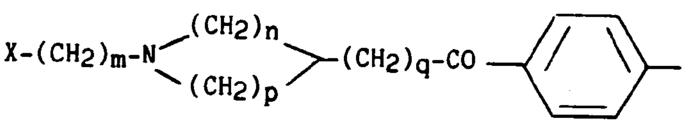

(V)

dans laquelle la signification de m, n, p, et q est identique à celle donnée pour la formule I selon la revendication 1 et X représente un groupe partant dont la définition est identique à celle donnée pour la formule générale II,
pour former les composés de formule I, selon la revendication 1,
soit:
l'on cyclise un dérivé de l'amino-4 imidazole avec un composé de formule VI:

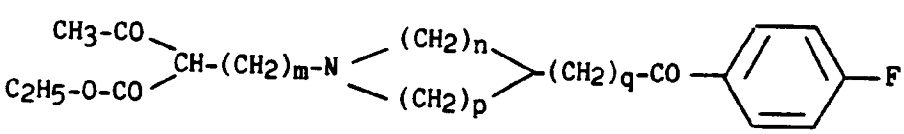

(VI)

dans laquelle m, n, p, et q ont la signification donnée pour la formule I, selon la revendication 1,
pour former les composés de la formule I, selon la revendication 1,

dans laquelle R représente un radical de formule B et m, n, p et q ont la signification indiquée pour la formule I,

lesquels ensuite

si l'on désire, sont séparés en leurs stéréoisomères possibles ou/et salifiés par un acide organique ou minéral, pharmaceutiquement acceptable, pour former les sels d'addition correspondants.

2. La cyano-8 {[(fluoro-4 benzoyl)-4 pipéridino]-2 éthyl}-3 hydroxy-4 méthyl-2 imidazo [1,5-a] pyrimidine, composé répondant à la formule générale I selon la revendication 1, ses isomères optiques et ses sels d'addition à un acide minéral ou organique pharmaceutiquement acceptable, lorsque préparée selon le procédé de la revendication 1 ou un procédé chimique équivalent.

3. La dioxo-2,4 {[(fluoro-4 phénacyl)-3 pyrrolidinyl-1]-2 éthyl}-3 tétrahydro-1,2,3,4 quinazoline, composé répondant à la formule générale I selon la revendication 1, ses isomères optiques et ses sels d'addition à un acide minéral ou organique pharmaceutiquement acceptable, lorsque préparée selon le procédé de la revendication 1 ou un procédé chimique équivalent.

4. La carbamoyl-8 {[(fluoro-4 phénacyl)-3 pyrrolidinyl-1]-2 éthyl}-3 hydroxy-4 méthyl-2 imidazo [1,5-a] pyrimidine, composé répondant à la formule générale I selon la revendication 1, ses isomères optiques et ses sels d'addition à un acide minéral ou organique pharmaceutiquement acceptable, lorsque préparée selon le procédé de la revendication 1 ou un procédé chimique équivalent.

5. La {[(fluoro-4 benzoyl)-4 pipéridino]-3 propyl}-1 (fluoro-2 benzyl)-4 pipérazine dione-2,6, composé répondant à la formule générale I selon la revendication 1, ses isomères optiques et ses sels d'addition à un acide minéral ou organique pharmaceutiquement acceptable, lorsque préparée selon le procédé de la revendication 1 ou un procédé chimique équivalent.

6. La {[(fluoro-4 benzoy)-4 pipéridino]-3 propyl}-1 (pyrimidinyl-2)-4 pipérazine, composé répondant à la formule générale I selon la revendication 1, ses isomères optiques et ses sels d'addition à un acide minéral ou organique pharmaceutiquement acceptable, lorsque préparée selon le procédé de la revendication 1 ou un procédé chimique équivalent.

**Revendications pour l'Etat contractant suivant : GR**

1. Procédé de préparation des composés de formule générale I:

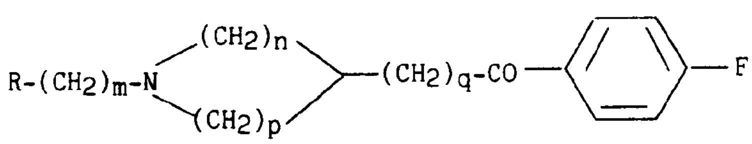

(I)

dans laquelle:

- m représente un nombre entier de 2 à 4,
- n et p identiques ou différents représentent chacun un nombre entier de 1 à 3, n et p ne représentant toutefois pas simultanément le nombre 3,
- q représente 0 ou 1

R représente :

. ou bien un groupement de formule (A):

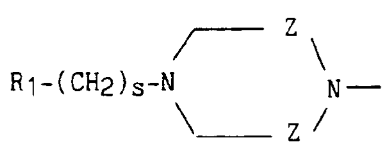

(A)

dans laquelle s représente un nombre entier de 0 à 4, Z représente un radical méthylène ou un radical carbonyle et $R_1$ représente soit un radical phényle (éventuellement substitué par un ou plusieurs atomes d'halogène, par un radical alcoyle inférieur linéaire ou ramifié contenant de 1 à 5 atomes de carbone ou un radical alcoxy inférieur contenant de 1 à 5 atomes de carbone) soit un radical diphénylméthylène (éventuellement substitué par un ou plusieurs atomes d'halogène, par un radical alcoyle inférieur ou un radical alcoxy inférieur), soit un cycle insaturé de cinq ou six chaînons comportant un ou deux atomes d'azote,

. ou bien un radical de formule (B) :

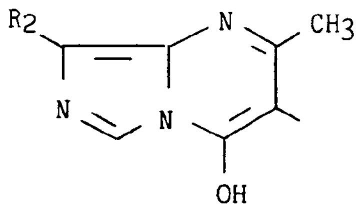

(B)

dans laquelle $R_2$ représente un radical carbamoyle, un radical cyano, un radical carboxy ou un radical alcoxycarbonyle contenant de 2 à 7 atomes de carbone,

. ou bien un radical dioxo-2,4 tétrahydro-1,2,3,4 quinazolinyle à la condition toutefois que, dans ce cas, n et p ne représentent pas simultanément le nombre 2,

. ou bien un radical oxo-1 phtalazinyle, à la condition que n et p ne représentent pas simultanément le nombre 2,

. ou bien un radical oxo-5 thiazolo [3,2-a] pyrimidinyle (éventuellement substitué par un ou plusieurs atomes d'halogène, par un radical alcoyle linéaire ou ramifié contenant de 1 à 5 atomes de carbone ou un radical alcoxy inférieur, contenant de 1 à 5 atomes de carbone), à la condition que n et p ne représentent pas simultanément le nombre 2,

. ou bien un groupement benzhydryloxy (dont les radicaux phényles sont éventuellement substitués par un ou plusieurs atomes d'halogène, par un radical alcoyle linéaire ou ramifié contenant de 1 à 5 atomes de carbone ou un radical alcoxy contenant de 1 à 5 atomes de carbone),

. ou bien un groupement de formule C :

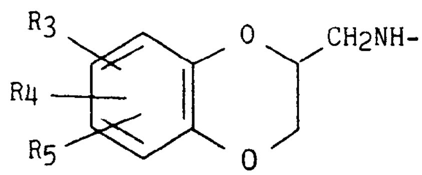

(C)

dans lequel $R_3$, $R_4$, $R_5$ identiques ou différents représentent chacun un atome d'halogène, un radical alcoxy de 1 à 5 atomes de carbone ou un radical alcoyle linéaire ou ramifié de 1 à 5 atomes de carbone,

leurs stéréoisomères possibles, et leurs sels d'addition à un acide minéral ou organique, pharmaceutiquement acceptable,

caractérisé en ce que :

soit l'on condense

ou bien

un composé de formule générale II :

$$R\text{-}(CH_2)_m\text{-}X \qquad (II)$$

dans laquelle R et m ont la même signification que pour la formule I selon la revendication 1 et X représente un groupe partant tel qu'un atome d'halogène, un radical mésyle ou un radical tosyle,

avec une amine de formule générale III

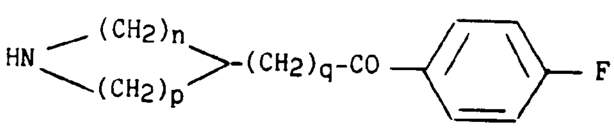

(III)

dans laquelle n, p, et q ont la signification indiquée pour la formule I, selon la revendication 1,

ou bien

que l'on condense un composé de formule générale IV:

**RH (IV)**

dans laquelle R a la même signification que pour la formule I, selon la revendication 1, avec un composé de formule V:

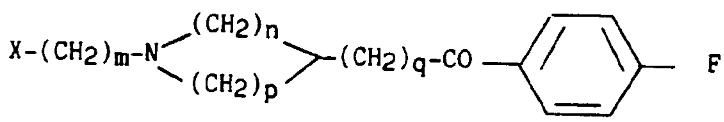

(V)

dans laquelle la signification de m, n, p, et q est identique à celle donnée pour la formule I selon la revendication 1 et X représente un groupe partant dont la définition est identique à celle donnée pour la formule générale II,
pour former les composés de formule I, selon la revendication 1,
soit:
l'on cyclise un dérivé de l'amino-4 imidazole avec un composé de formule VI:

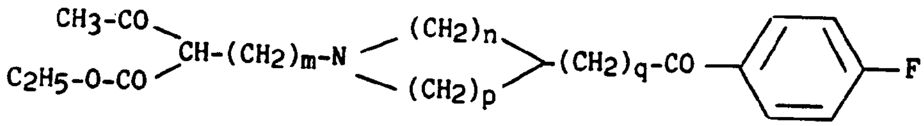

(VI)

dans laquelle m, n, p, et q ont la signification donnée pour la formule I, selon la revendication 1,
pour former les composés de la formule I, selon la revendication 1,
dans laquelle R représente un radical de formule B et m, n, p et q ont la signification indiquée pour la formule I,
lesquels ensuite
si l'on désire, sont séparés en leurs stéréoisomères possibles ou/et salifiés par un acide organique ou minéral, pharmaceutiquement acceptable, pour former les sels d'addition correspondants.

2. La cyano-8 {[(fluoro-4 benzoyl)-4 pipéridino]-2 éthyl}-3 hydroxy-4 méthyl-2 imidazo [1,5-a] pyrimidine, composé répondant à la formule générale I selon la revendication 1, ses isomères optiques et ses sels d'addition à un acide minéral ou organique pharmaceutiquement acceptable, lorsque préparée selon le procédé de la revendication 1 ou un procédé chimique équivalent.

3. La dioxo-2,4 {[(fluoro-4 phénacyl)-3 pyrrolidinyl-1]-2 éthyl}-3 tétrahydro-1,2,3,4 quinazoline, composé répondant à la formule générale I selon la revendication 1, ses isomères optiques et ses sels d'addition à un acide minéral ou organique pharmaceutiquement acceptable, lorsque préparée selon le procédé de la revendication 1 ou un procédé chimique équivalent.

4. La carbamoyl-8 {[(fluoro-4 phénacyl)-3 pyrrolidinyl-1]-2 éthyl}-3 hydroxy-4 méthyl-2 imidazo [1,5-a] pyrimidine, composé répondant à la formule générale I selon la revendication 1, ses isomères optiques et ses sels d'addition à un acide minéral ou organique pharmaceutiquement acceptable, lorsque préparée selon le procédé de la revendication 1 ou un procédé chimique équivalent.

5. La {[(fluoro-4 benzoyl)-4 pipéridino]-3 propyl}-1 (fluoro-2 benzyl)-4 pipérazine dione-2,6, composé répondant à la formule générale I selon la revendication 1, ses isomères optiques et ses sels d'addition à un acide minéral ou organique pharmaceutiquement acceptable, lorsque préparée selon le procédé de la revendication 1 ou un procédé chimique équivalent.

6. La {[(fluoro-4 benzoy)-4 pipéridino]-3 propyl}-1 (pyrimidinyl-2)-4 pipérazine, composé répondant à la formule générale I selon la revendication 1, ses isomères optiques et ses sels d'addition à un acide minéral ou organique pharmaceutiquement acceptable, lorsque préparée selon le procédé de la revendication 1 ou un procédé chimique équivalent.

## Patentansprüche

### Patentansprüche für folgende Vertragsstaaten : AT, BE, CH, DE, DK, FR, GB, IT, LI, LU, NL, SE

**1.** Verbindungen der allgemeinen Formel I:

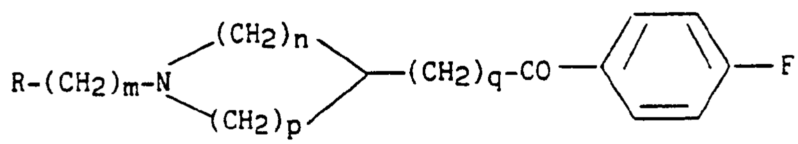

(I)

in der

- m eine ganze Zahl mit einem Wert von 2 bis 4,
- n und p, die gleichartig oder verschieden sein können, jeweils eine ganze Zahl mit einem Wert von 1 bis 3, wobei n und p nicht gleichzeitig die Zahl 3 bedeuten,
- q 0 oder 1 und

R:

- entweder eine Gruppe der Formel (A):

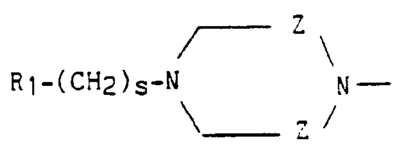

(A)

in der s eine ganze Zahl mit einem Wert von 0 bis 4, Z eine Methylengruppe oder eine Carbonylgruppe und $R_1$ entweder eine Phenylgruppe (die gegebenenfalls durch eines oder mehrere Halogenatome, durch eine geradkettige oder verzweigtkettige Niedrigalkylgruppe mit 1 bis 5 Kohlenstoffatomen oder eine Niedrigalkoxygruppe mit 1 bis 5 Kohlenstoffatomen substituiert ist) oder eine Diphenylmethylengruppe (die gegebenenfalls durch eines oder mehrere Halogenatome, durch eine Niedrigalkylgruppe oder eine Niedrigalkoxygruppe substituiert ist) oder einen ungesättigten Ring mit 5 oder 6 Kettengliedern, der ein oder zwei Stickstoffatome enthält, darstellen,

- oder eine Gruppe der Formel (B):

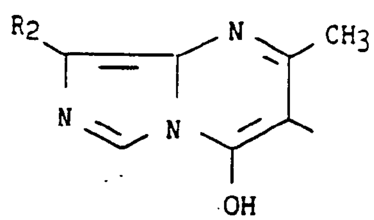

(B)

in der $R_2$ eine Carbamoylgruppe, eine Cyanogruppe, eine Carboxygruppe oder eine Alkoxycarbonylgruppe mit 2 bis 7 Kohlenstoffatomen darstellt,

- oder eine 2,4-Dioxo-1,2,3,4-tetrahydro-chinazolinylgruppe,mit der Maßgabe, daß in diesem Fall n und p nicht gleichzeitig die Zahl 2 bedeuten,
- oder eine 1-Oxophthalazinylgruppe, mit der Maßgabe, daß n und p nicht gleichzeitig die Zahl 2 bedeuten,
- oder eine 5-Oxo-thiaozolo[3,2-a]pyrimidinylgruppe (die gegebenenfalls durch eines oder mehrere Halogenatome, durch eine geradkettige oder verzweigte Alkylgruppe mit 1 bis 5 Kohlenstoffatomen oder eine Niedrigalkoxygruppe mit 1 bis 5 Kohlenstoffatomen substituiert ist), mit der Maßgabe, daß n und p nicht gleichzeitig die Zahl 2 bedeuten,
- oder eine Benzhydryloxygruppe (deren Phenylgruppen gegebenenfalls durch eines oder mehrere Halogenatome, durch eine geradkettige oder verzweigte Alkylgruppe mit 1 bis 5 Kohlenstoffatomen oder eine Alkoxygruppe mit 1 bis 5 Kohlenstoffatomen substituiert sind),
- oder eine Gruppe der Formel (C):

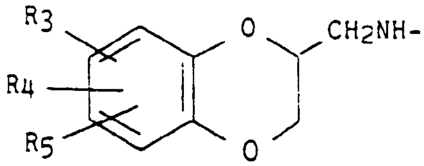

(C)

in der $R_3$, $R_4$ und $R_5$, die gleich oder verschieden sein können, jeweils ein Halogenatom, eine Alkoxygruppe mit 1 bis 5 Kohlenstoffatomen oder eine geradkettige oder verzweigte Alkylgruppe mit 1 bis 5 Kohlenstoffatomen darstellen, bedeuten,

deren mögliche Stereoisomeren und deren Additionssalze mit einer anorganischen oder organischen, pharmazeutisch annehmbaren Säure.

2. 8-Cyano-3-{2-[4-(4-fluor-benzoyl)-piperidino]-ethyl}-4-hydroxy-2-methyl-imidazo[1,5-a]pyrimidin, Verbindung der allgemeinen Formel I nach Anspruch 1 und deren Additionssalze mit einer anorganischen oder organischen, pharmazeutisch annehmbaren Säure.

3. 2.4-Dioxo-3-{2-{3-(4-fluor-phenacyl}-pyrrolidin-1-yl]-ethyl}-1,2,3,4-tetrahydro-chinazolin, Verbindung der allgemeinen Formel I nach Anspruch 1, dessen optische Isomeren und deren Additionssalze mit einer anorganischen oder organischen, pharmazeutisch annehmbaren Säure.

4. 8-Carbamoyl-3-{2-[3-(4-fluor-phenacyl)-pyrrolidin-1-yl]-ethyl}-4-hydroxy-2-methyl-imidazo[1,5-a]pyrimi din, Verbindung der allgemeinen Formel I nach Anspruch 1, und deren Additionssalze mit einer anorganischen oder organischen, pharmazeutisch annehmbaren Säure.

5. 1-(3-[4-{4-Fluor-benzoyl)-piperidino]-propyl}-4-(2-fluor-benzyl)-pipera- zin-2,6-dion, Verbindung der allgemeinen Formel I nach Anspruch 1, und deren Additionssalze mit einer anorganischen oder organischen, pharmazeutisch annehmbaren Säure.

6. 1-{3-[4-(4-Fluor-benzoyl)-piperidino]-propyl}-4-(pyrimidin-2-yl)-piperazin, Verbindung der allgemeinen Formel I nach Anspruch 1, und deren Additionssalze mit einer anorganischen oder organischen, pharmazeutisch annehmbaren Säure.

7. Verfahren zur Herstellung der Verbindung der allgemeinen Formel I nach Anspruch 1, **dadurch gekennzeichnet**, daß man

**entweder**

eine Verbindung der allgemeinen Formel II:

$$R\text{-}(CH_2)_m\text{-}X \qquad (II)$$

in der R und m die bezüglich der Formel I in Anspruch 1 angegebenen Bedeutungen besitzen und X eine austretende Gruppe, wie ein Halogenatom, eine Mesylgruppe oder eine Tosylgruppe, darstellt,

mit einem Amin der allgemeinen Formel III:

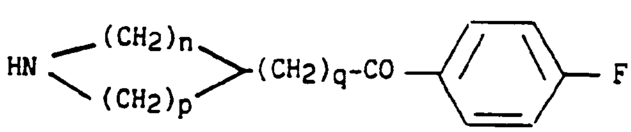

(III)

in der n, p und q die bezüglich der Formel I in Anspruch 1 angegebenen Bedeutungen besitzen, kondensiert,

<u>oder</u>

eine Verbindung der allgemeinen Formel IV

$$RH \qquad (IV)$$

in der R die bezüglich der Formel I in Anspruch 1 angegebenen Bedeutungen besitzt,

mit einer Verbindung der Formel V:

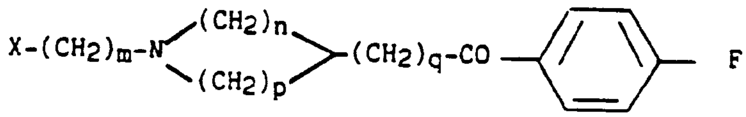

(V)

in der die Bedeutung von m, n, p und q identisch ist mit der bezüglich der Formel I in Anspruch 1 angegebenen und X eine austretende Gruppe darstellt, deren Definition identisch ist der für die allgemeine Formel II angegebenen, kondensiert zur Bildung der Verbindungen der Formel I nach Anspruch 1,
**oder**
ein 4-Amino-imidazolderivat mit einer Verbindung der Formel VI:

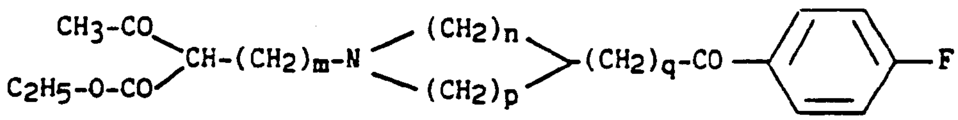

(VI)

in der m, n, p und q die bezüglich der Formel I in Anspruch 1 angegebenen Bedeutungen besitzen, cyclisiert zur Bildung der Verbindungen der Formel I nach Anspruch 1, worin R eine Gruppe der Formel B darstellt und m, n, p und q die bezüglich der Formel I angegebenen Bedeutungen besitzen,
welche man
**gewünschtenfalls** in ihre möglichen Stereoisomeren auftrennt und/oder mit einer organischen oder anorganischen, pharmazeutisch annehmbaren Säure zur Bildung der entsprechenden Säureadditionssalze in die Salze überführt.

8. Pharmazeutische Zubereitung enthaltend als Wirkstoff eine Verbindung nach einem der Ansprüche 1 bis 6 in Kombination oder in Mischung mit einem inerten, nicht-toxischen, pharmazeutisch annehmbaren Trägermaterial oder Bindemittel.

9. Pharmazeutische Zubereitung nach Anspruch 8, enthaltend den Wirkstoff in einer Dosis von 0,5 bis 100 mg.

10. Pharmazeutische Zubereitung nach den Ansprüchen 8 und 9, enthaltend als Wirkstoff mindestens eine Verbindung nach den Ansprüchen 1 bis 6 zur Behandlung von Krankheiten, welche $\alpha 1$ -adrenergische Antagonisten, SerotoninAntagonisten und Histamin-Antagonisten erfordern.

**Patentansprüche für folgenden Vertragsstaat : ES**

1. Verfahren zur Herstellung von Verbindungen der allgemeinen Formel I:

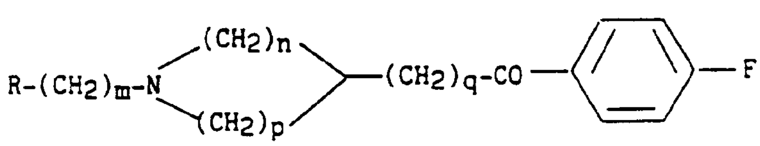

(I)

in der
- m eine ganze Zahl mit einem Wert von 2 bis 4,
- n und p. die gleichartig oder verschieden sein können, jeweils eine ganze Zahl mit einem Wert von 1 bis 3, wobei n und p nicht gleichzeitig die Zahl 3 bedeuten,
- q 0 oder 1 und

R:
- entweder eine Gruppe der Formel (A):

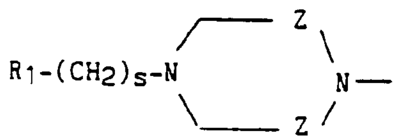

(A)

in der s eine ganze Zahl mit einem Wert von 0 bis 4, Z eine Methylengruppe oder eine Carbonylgruppe und $R_1$ entweder eine Phenylgruppe (die gegebenenfalls durch eines oder mehrere Halogenatome, durch eine geradkettige oder verzweigtkettige Niedrigalkylgruppe mit 1 bis 5 Kohlenstoffatomen oder eine Niedrigalkoxygruppe mit 1 bis 5 Kohlenstoffatomen substituiert ist) oder eine Diphenylmethylengruppe (die gegebenenfalls durch eines oder mehrere Halogenatome, durch eine Niedrigalkylgruppe oder eine Niedrigalkoxygruppe substituiert ist) oder einen ungesättigten Ring mit 5 oder 6 Kettengliedern, der ein oder zwei Stickstoffatome enthält, darstellen,

- oder eine Gruppe der Formel (B):

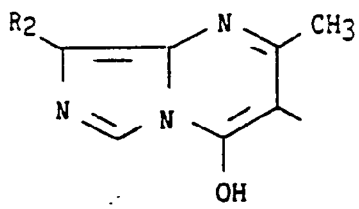

(B)

in der $R_2$ eine Carbamoylgruppe, eine Cyanogruppe, eine Carboxygruppe oder eine Alkoxycarbonylgruppe mit 2 bis 7 Kohlenstoffatomen darstellt,

- oder eine 2,4-Dioxo-1,2,3,4-tetrahydro-chinazolinylgruppe, mit der Maßgabe, daß in diesem Fall n und p nicht gleichzeitig die Zahl 2 bedeuten,
- oder eine 1-Oxophthalazinylgruppe, mit der Maßgabe, daß n und p nicht gleichzeitig die Zahl 2 bedeuten,
- oder eine 5-Oxo-thiaozolo[3,2-a]pyrimidinylgruppe (die gegebenenfalls durch eines oder mehrere Halogenatome, durch eine geradkettige oder verzweigte Alkylgruppe mit 1 bis 5 Kohlenstoffatomen oder eine Niedrigalkoxygruppe mit 1 bis 5 Kohlenstoffatomen substituiert ist), mit der Maßgabe, daß n und p nicht gleichzeitig die Zahl 2 bedeuten,
- oder eine Benzhydryloxygruppe (deren Phenylgruppen gegebenenfalls durch eines oder mehrere Halogenatome, durch eine geradkettige oder verzweigte Alkylgruppe mit 1 bis 5 Kohlenstoffatomen oder eine Alkoxygruppe mit 1 bis 5 Kohlenstoffatomen substituiert sind),
- oder eine Gruppe der Formel (C):

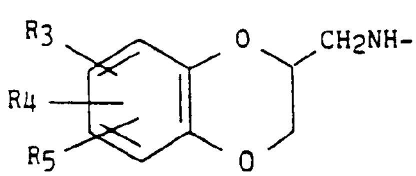

(C)

in der $R_3$, $R_4$ und $R_5$, die gleich oder verschieden sein können, jeweils ein Halogenatom, eine Alkoxygruppe mit 1 bis 5 Kohlenstoffatomen oder eine geradkettige oder verzweigte Alkylgruppe mit 1 bis 5 Kohlenstoffatomen darstellen, bedeuten,

von deren möglichen Stereoisomeren und deren Additionssalzen mit einer anorganischen oder organischen, pharmazeutisch annehmbaren Säure, **dadurch gekennzeichnet**, daß man

**entweder**

eine Verbindung der allgemeinen Formel II:

$$R\text{-}(CH_2)_m\text{-}X \qquad (II)$$

in der R und m die bezüglich der Formel I angegebenen Bedeutungen besitzen und X eine austretende Gruppe, wie ein Halogenatom, eine Mesylgruppe oder eine Tosylgruppe, darstellt,

mit einem Amin der allgemeinen Formel III:

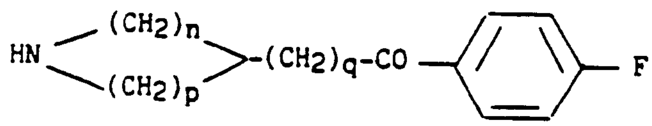 (III)

in der n, p und q die bezüglich der Formel I angegebenen Bedeutungen besitzen, kondensiert,
<u>oder</u>
eine Verbindung der allgemeinen Formel IV

RH (IV)

in der R die bezüglich der Formel I angegebenen Bedeutungen besitzt, mit einer Verbindung der Formel V:

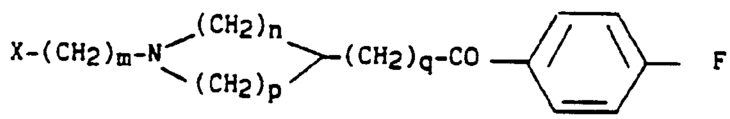 (V)

in der die Bedeutung von m, n, p und q identisch ist mit der bezüglich der Formel I angegebenen und X eine austretende Gruppe darstellt, deren Definition identisch ist der für die allgemeine Formel II angegebenen, kondensiert zur Bildung der Verbindungen der Formel I,
**oder**
ein 4-Amino-imidazolderivat mit einer Verbindung der Formel VI:

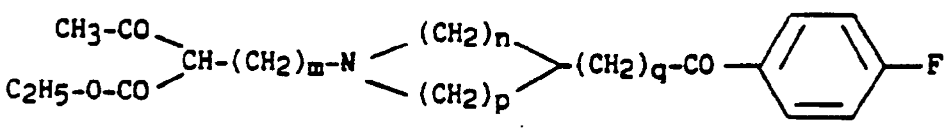 (VI)

in der m, n, p und q die bezüglich der Formel I angegebenen Bedeutungen besitzen, cyclisiert zur Bildung der Verbindungen der Formel I, worin R eine Gruppe der Formel B darstellt und m, n, p und q die bezüglich der Formel I angegebenen Bedeutungen besitzen,
welche man
**gewünschtenfalls** in ihre möglichen Stereoisomeren auftrennt und/oder mit einer organischen oder anorganischen, pharmazeutisch annehmbaren Säure zur Bildung der entsprechenden Säureadditionssalze in die Salze überführt.

**2.** 8-Cyano-3-{2-[4-(4-fluor-benzoyl)-piperidino]-ethyl}-4-hydroxy-2-methyl-imidazo[1.5-a]pyrimidin, Verbindung der allgemeinen Formel I nach Anspruch 1 und deren Additionssalze mit einer anorganischen oder organischen, pharmazeutisch annehmbaren Säure oder einem chemisch äquivalenten Verfahren.

**3.** 2,4-Dioxo-3-{2-[3-{4-fluor-phenacyl}-pyrrolidin-1-yl]-ethyl}-1,2,3,4-tetrahydro-chinazolin, Verbindung der allgemeinen Formel 1 nach Anspruch 1, dessen optische Isomeren und deren Additionssalze mit einer anorganischen oder organischen, pharmazeutisch annehmbaren Säure oder einem chemisch äquivalenten Verfahren.

**4.** 8-Carbamoyl-3-{2-[3-(4-fluor-phenacyl)-pyrrolidin-1-yl]-ethyl}-4-hydroxy-2-methyl-imidazo[1,5-a]pyrimi din, Verbindung der allgemeinen Formel I nach Anspruch 1, und deren Additionssalze mit einer anorganischen oder organischen, pharmazeutisch annehmbaren Säure oder einem chemisch äquivalen- ten Verfahren.

**5.** 1-{3-[4-(4-Fluor-benzoyl)-piperidino]-propyl}-4-(2-fluor-benzyl)-piperazin-2,6-dion, Verbindung der allgemeinen Formel I nach Anspruch 1, und deren Additionssalze mit einer anorganischen oder organischen, pharmazeutisch annehmbaren Säure oder einem chemisch äquivalenten Verfahren.

**6.** 1-{3-[4-(4-Fluor-benzoyl)-piperidino]-propyl}-4-(pyrimidin-2-yl)-piperazin, Verbindung der allgemeinen

Formel I nach Anspruch 1, und deren Additionssalze mit einer anorganischen oder organischen, pharmazeutisch annehmbaren Säure, erhalten nach dem Verfahren nach Anspruch 1 oder einem chemisch äquivalenten Verfahren.

**Patentansprüche für den Vertragsstaat : GR**

1. Verfahren zur Herstellung von Verbindungen der allgemeinen Formel I:

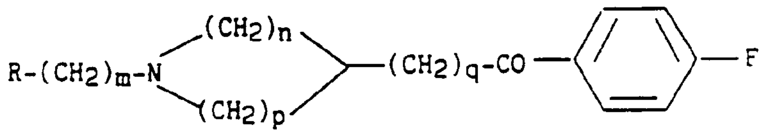

(I)

in der
- m eine ganze Zahl mit einem Wert von 2 bis 4,
- n und p, die gleichartig oder verschieden sein können, jeweils eine ganze Zahl mit einem Wert von 1 bis 3, wobei n und p nicht gleichzeitig die Zahl 3 bedeuten,
- q 0 oder 1 und

R:
- entweder eine Gruppe der Formel (A):

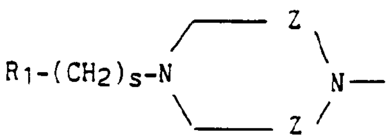

(A)

in der s eine ganze Zahl mit einem Wert von 0 bis 4. Z eine Methylengruppe oder eine Carbonylgruppe und $R_1$ entweder eine Phenylgruppe (die gegebenenfalls durch eines oder mehrere Halogenatome, durch eine geradkettige oder verzweigtkettige Niedrigalkylgruppe mit 1 bis 5 Kohlenstoffatomen oder eine Niedrigalkoxygruppe mit 1 bis 5 Kohlenstoffatomen substituiert ist) oder eine Diphenylmethylengruppe (die gegebenenfalls durch eines oder mehrere Halogenatome, durch eine Niedrigalkylgruppe oder eine Niedrigalkoxygruppe substituiert ist) oder einen ungesättigten Ring mit 5 oder 6 Kettengliedern, der ein oder zwei Stickstoffatome enthält, darstellen.
- oder eine Gruppe der Formel (B):

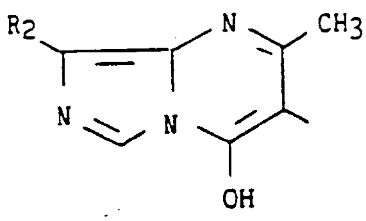

(B)

in der $R_2$ eine Carbamoylgruppe, eine Cyanogruppe, eine Carboxygruppe oder eine Alkoxycarbonylgruppe mit 2 bis 7 Kohlenstoffatomen darstellt,
- oder eine 2,4-Dioxo-1,2,3,4-tetrahydro-chinazolinylgruppe, mit der Maßgabe, daß in diesem Fall n und p nicht gleichzeitig die Zahl 2 bedeuten,
- oder eine 1-Oxophthalazinylgruppe, mit der Maßgabe, daß n und p nicht gleichzeitig die Zahl 2 bedeuten,
- oder eine 5-Oxo-thiazolo[3,2-a]pyrimidinylgruppe (die gegebenenfalls durch eines oder mehrere Halogenatome, durch eine geradkettige oder verzweigte Alkylgruppe mit 1 bis 5 Kohlenstoffatomen oder eine Niedrigalkoxygruppe mit 1 bis 5 Kohlenstoffatomen substituiert ist), mit der Maßgabe, daß n und p nicht gleichzeitig die Zahl 2 bedeuten,
- oder eine Benzhydryloxygruppe (deren Phenylgruppen gegebenenfalls durch eines oder mehrere Halogenatome, durch eine geradkettige oder verzweigte Alkylgruppe mit 1 bis 5 Kohlenstoffatomen oder eine Alkoxygruppe mit 1 bis 5 Kohlenstoffatomen substituiert sind),

- oder eine Gruppe der Formel (C):

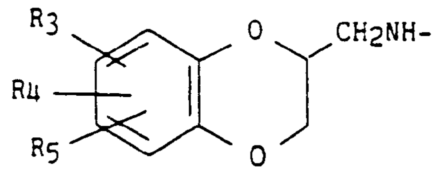

(C)

in der $R_3$, $R_4$ und $R_5$, die gleich oder verschieden sein können, jeweils ein Halogenatom, eine Alkoxygruppe mit 1 bis 5 Kohlenstoffatomen oder eine geradkettige oder verzweigte Alkylgruppe mit 1 bis 5 Kohlenstoffatomen darstellen, bedeuten,

von deren möglichen Stereoisomeren und deren Additionssalzen mit einer anorganischen oder organischen, pharmazeutisch annehmbaren Säure, **dadurch gekennzeichnet,** daß man

**entweder**

eine Verbindung der allgemeinen Formel II:

$$R\text{-}(CH_2)_m\text{-}X \qquad (II)$$

in der R und m die bezüglich der Formel I angegebenen Bedeutungen besitzen und X eine austretende Gruppe. wie ein Halogenatom, eine Mesylgruppe oder eine Tosylgruppe, darstellt,

mit einem Amin der allgemeinen Formel III:

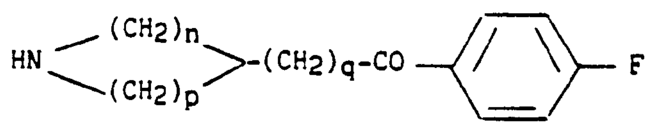

(III)

in der n, p und q die bezüglich der Formel I angegebenen Bedeutungen besitzen, kondensiert,

oder

eine Verbindung der allgemeinen Formel IV

$$RH \qquad (IV)$$

in der R die bezüglich der Formel I angegebenen Bedeutungen besitzt, mit einer Verbindung der Formel V:

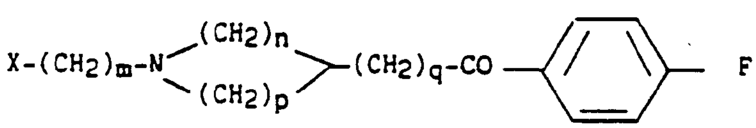

(V)

in der die Bedeutung von m, n, p und q identisch ist mit der bezüglich der Formel I angegebenen und X eine austretende Gruppe darstellt, deren Definition identisch ist der für die allgemeine Formel II angegebenen, kondensiert zur Bildung der Verbindungen der Formel I,

**oder**

ein 4-Amino-imidazolderivat mit einer Verbindung der Formel VI:

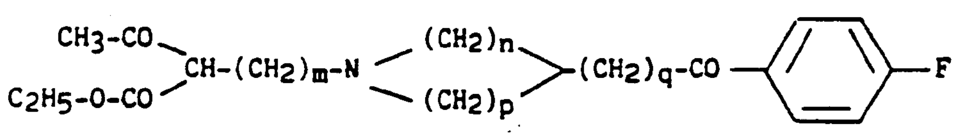

(VI)

in der m, n, p und q die bezüglich der Formel I angegebenen Bedeutungen besitzen, cyclisiert zur Bildung der Verbindungen der Formel I, worin R eine Gruppe der Formel B darstellt und m, n, p und q die bezüglich der Formel I angegebenen Bedeutungen besitzen,

welche man

**gewünschtenfalls** in ihre möglichen Stereoisomeren auftrennt und/oder mit einer organischen oder anorganischen, pharmazeutisch annehmbaren Säure zur Bildung der entsprechenden Säureadditionssalze

in die Salze überführt.

2. 8-Cyano-3-{2-[4-(4-fluor-benzoyl)-piperidino]-ethyl}-4-hydroxy-2-methyl-imidazo[1,5-a]pyrimidin, Verbindung der allgemeinen Formel I nach Anspruch 1 und deren Additionssalze mit einer anorganischen oder organischen, pharmazeutisch annehmbaren Säure oder einem chemisch äquivalenten Verfahren.

3. 2,4-Dioxo-3-{2-[3-{4-fluor-phenacyl}-pyrrolidin-1-yl]-ethyl}-1,2,3,4-tetrahydro-chinazolin, Verbindung der allgemeinen Formel I nach Anspruch 1, dessen optische Isomeren und deren Additionssalze mit einer anorganischen oder organischen, pharmazeutisch annehmbaren Säure oder einem chemisch äquivalenten Verfahren.

4. 8-Carbamoyl-3-{2-[3-(4-fluor-phenacyl)-pyrrolidin-1-yl]-ethyl}-4-hydroxy-2-methyl-imidazo[1,5-a]pyrimi din, Verbindung der allgemeinen Formel I nach Anspruch 1, und deren Additionssalze mit einer anorganischen oder orga nischen, pharmazeutisch annehmbaren Säure oder einem chemisch äquivalenten Verfahren.

5. 1-{3-[4-{4-Fluor-benzoyl)-piperidino]-propyl}-4-(2-fluor-benzyl}-piperazin-2,6-dion, Verbindung der allgemeinen Formel I nach Anspruch 1, und deren Additionssalze mit einer anorganischen oder organischen, pharmazeutisch annehmbaren Säure oder einem chemisch äquivalenten Verfahren.

6. 1-{3-[4-(4-Fluor-benzoyl)-piperidino]-propyl}-4-(pyrimidin-2-yl)-piperazin, Verbindung der allgemeinen Formel I nach Anspruch 1, und deren Additionssalze mit einer anorganischen oder organischen, pharmazeutisch annehmbaren Säure, erhalten nach dem Verfahren nach Anspruch 1 oder einem chemisch äquivalenten Verfahren.

## Claims

### Claims for the following Contracting States : AT, BE, CH, DE, DK, FR, GB, IT, LI, LU, NL, SE

1. Compounds of the general formula I:

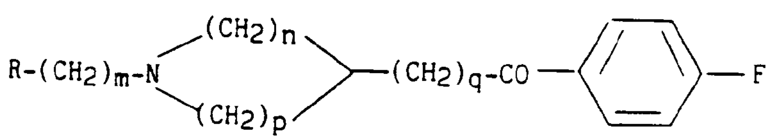

(I)

wherein:

- m represents an integer from 2 to 4,
- each of n and p, which may be identical or different, represents an integer from 1 to 3, but n and p do not simultaneously represent the number 3,
- q represents 0 or 1, and

R represents:

. a grouping of formula (A):

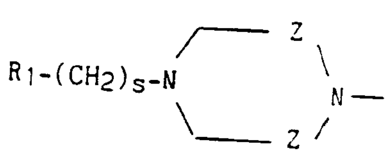

(A)

wherein s represents an integer from 0 to 4, Z represents a methylene radical or a carbonyl radical and $R_1$ represents either a phenyl radical (optionally substituted by one or more halogen atoms, by a linear or branched lower alkyl radical containing from 1 to 5 carbon atoms or a lower alkoxy radical containing from 1 to 5 carbon atoms) or a diphenylmethylene radical (optionally substituted by one or more halogen atoms, by a lower alkyl radical or a lower alkoxy radical), or an unsaturated ring having five or six ring members and comprising one or two nitrogen atoms,

. or a radical of formula (B):

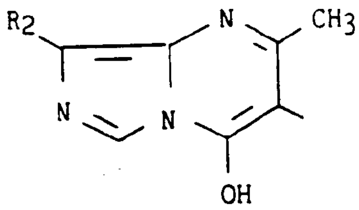

(B)

wherein $R_2$ represents a carbamoyl radical, a cyano radical, a carboxy radical or an alkoxycarbonyl radical containing from 2 to 7 carbon atoms,

. or a 2,4-dioxo-1,2,3,4-tetrahydroquinazolinyl radical with the proviso that, in that case, n and p do not simultaneously represent the number 2,

. or a 1-oxophthalazinyl radical, with the proviso that n and p do not simultaneously represent the number 2,

. or a 5-oxothiazolo[3,2-a]pyrimidinyl radical (optionally substituted by one or more halogen atoms, by a linear or branched alkyl radical containing from 1 to 5 carbon atoms or a lower alkoxy radical containing from 1 to 5 carbon atoms), with the proviso that n and p do not simultaneously represent the number 2,

. or a benzhydryloxy grouping (the phenyl radicals of which are optionally substituted by one or more halogen atoms, by a linear or branched alkyl radical containing from 1 to 5 carbon atoms or an alkoxy radical containing from 1 to 5 carbon atoms),

. or a grouping of the formula C:

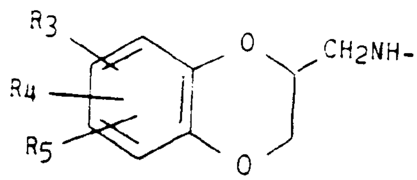

(C)

wherein each of $R_3$, $R_4$ and $R_5$, which may be identical or different, represents a halogen atom, an alkoxy radical having from 1 to 5 carbon atoms or a linear or branched alkyl radical having from 1 to 5 carbon atoms,

their possible stereoisomers, and their addition salts with a pharmaceutically acceptable mineral or organic acid.

2. 8-cyano-3-{2-[4-(4-fluorobenzoyl)-piperidino]-ethyl}4-hydroxy-2-methylimidazo[1,5-a]pyrimidine, a compound corresponding to the general formula I according to claim 1 and its addition salts with a pharmaceutically acceptable mineral or organic acid.

3. 2,4-dioxo-3-{2-[3-(4-fluorophenacyl)-1-pyrrolidinyl]ethyl}-1,2,3 ,4-tetrahydroquinazoline, a compound corresponding to the general formula I according to claim 1, its optical isomers and its addition salts with a pharmaceutically acceptable mineral or organic acid.

4. 8-carbamoyl-3-{2-[3-(4-fluorophenacyl)-1-pyrrolidinyl]-ethyl}-4-hydroxy-2-methylimidazo[1,5-a]pyrimidine, a compound corresponding to the general formula I according to claim 1 and its addition salts with a pharmaceutically acceptable mineral or organic acid.

5. 1-{3-[4-(4-fluorobenzoyl)-piperidinol-propyl}-4-(2-fluorobenzyl)-piperazine-2,6-dione, a compound corresponding to the general formula I according to claim 1 and its addition salts with a pharmaceutically acceptable mineral or organic acid.

6. 1-{3-[4-(4-fluorobenzoyl)-piperidino]-propyl}-4-(2-pyrimidinyl)-piperazine, a compound corresponding to the general formula I according to claim 1 and its addition salts with a pharmaceutically acceptable mineral

or organic acid.

7. Process for the preparation of the compounds of the general formula I according to claim 1, characterised in that:
either:
a compound of the general formula II:

$$R\text{-}(CH_2)_m\text{-}X \qquad (II)$$

wherein R and m are as defined for formula I according to claim 1 and X represents a leaving group, such as a halogen atom, a mesyl radical or a tosyl radical, is condensed with an amine of the general formula III

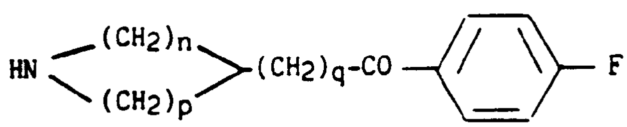

(III)

wherein n, p and q are as defined for formula I according to claim 1,
or
a compound of the general formula IV:

$$RH \qquad (IV)$$

wherein R is as defined for formula I according to claim 1,
is condensed with a compound of formula V:

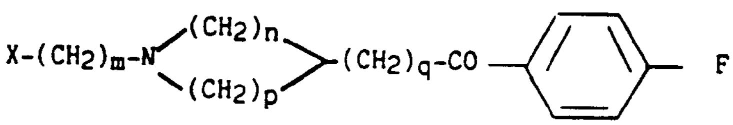

(V)

wherein m, n, p and q are as defined for formula I according to claim 1 and X represents a leaving group which is as defined for the general formula II, to form the compounds of formula I according to claim 1,
or:
a 4-aminoimidazole compound is cyclised with a compound of formula VI:

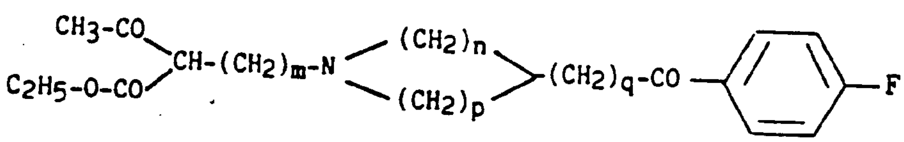

(VI)

wherein m, n, p and q are as defined for formula I according to claim 1,
to form the compounds of formula I according to claim 1 wherein R represents a radical of formula B and m, n, p and q are as defined for formula I,
which are then,
if desired, separated into their possible stereoisomers and/or converted into salts with a pharmaceutically acceptable organic or mineral acid to form the corresponding addition salts.

8. Pharmaceutical composition containing as active ingredient a compound according to any one of claims 1 to 6 in association or in admixture with a pharmaceutically acceptable inert non-toxic excipient or carrier.

9. Pharmaceutical composition according to claim 8, containing the active ingredient in an amount of from 0.5 to 100 mg.

10. Pharmaceutical composition according to claims 8 and 9 containing as active ingredient at least one compound according to claims 1 to 6 for use in the treatment of disorders necessitating $\alpha_1$-adrenergic antagonists of serotonin and histamine.

**Claims for the following Contracting State : ES**

1. Process for the preparation of the compounds of the general formula I:

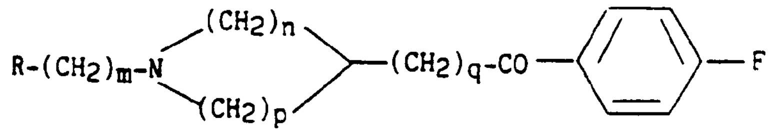

(I)

wherein:

- m represents an integer from 2 to 4,
- each of n and p, which may be identical or different, represents an integer from 1 to 3, but n and p do not simultaneously represent the number 3,
- q represents 0 or 1,

and

R represents:

. a grouping of formula (A):

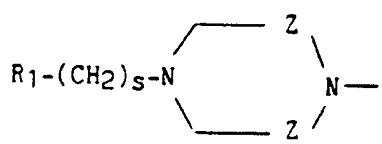

(A)

wherein s represents an integer from 0 to 4, Z represents a methylene radical or a carbonyl radical and $R_1$ represents either a phenyl radical (optionally substituted by one or more halogen atoms, by a linear or branched lower alkyl radical containing from 1 to 5 carbon atoms or a lower alkoxy radical containing from 1 to 5 carbon atoms) or a diphenylmethylene radical (optionally substituted by one or more halogen atoms, by a lower alkyl radical or a lower alkoxy radical), or an unsaturated ring having five or six ring members and comprising one or two nitrogen atoms,

. or a radical of formula (B):

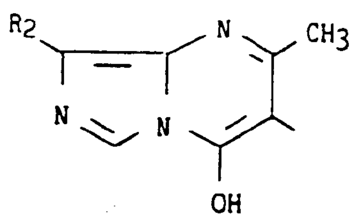

(B)

wherein $R_2$ represents a carbamoyl radical, a cyano radical, a carboxy radical or an alkoxycarbonyl radical containing from 2 to 7 carbon atoms,

. or a 2,4-dioxo-1,2,3,4-tetrahydroquinazolinyl radical with the proviso that, in that case, n and p do not simultaneously represent the number 2,

. or a 1-oxophthalazinyl radical, with the proviso that n and p do not simultaneously represent the number 2,

. or a 5-oxothiazolo[3,2-a]pyrimidinyl radical (optionally substituted by one or more halogen atoms, by a linear or branched alkyl radical containing from 1 to 5 carbon atoms or a lower alkoxy radical containing from 1 to 5 carbon atoms), with the proviso that n and p do not simultaneously represent the number 2,

. or a benzhydryloxy grouping (the phenyl radicals of which are optionally substituted by one or more halogen atoms, by a linear or branched alkyl radical containing from 1 to 5 carbon atoms or an alkoxy radical containing from 1 to 5 carbon atoms),

. or a grouping of the formula C:

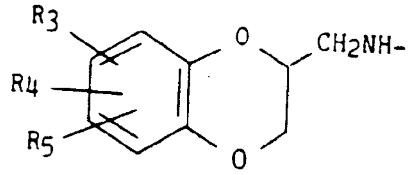

(C)

wherein each of $R_3$, $R_4$ and $R_5$, which may be identical or different, represents a halogen atom, an alkoxy radical having from 1 to 5 carbon atoms or a linear or branched alkyl radical having from 1 to 5 carbon atoms,

their possible stereoisomers, and their addition salts with a pharmaceutically acceptable mineral or organic acid,

characterised in that:

either:

a compound of the general formula II:

$$R\text{-}(CH_2)_m\text{-}X \qquad (II)$$

wherein R and m are as defined for formula I according to claim 1 and X represents a leaving group, such as a halogen atom, a mesyl radical or a tosyl radical, is condensed with an amine of the general formula III

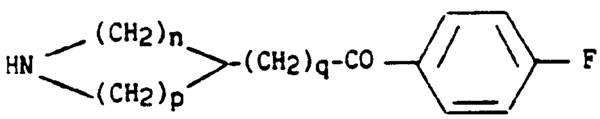

(III)

wherein n, p and q are as defined for formula I according to claim 1,

or

a compound of the general formula IV:

$$RH \qquad (IV)$$

wherein R is as defined for formula I according to claim 1,

is condensed with a compound of formula V:

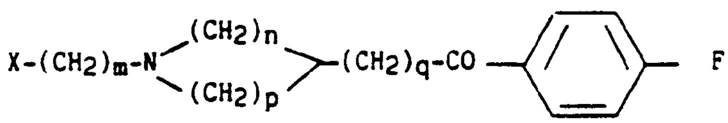

(V)

wherein m, n, p and q are as defined for formula I according to claim 1 and X represents a leaving group which is as defined for the general formula II, to form the compounds of formula I according to claim 1,

or:

a 4-aminoimidazole compound is cyclised with a compound of formula VI:

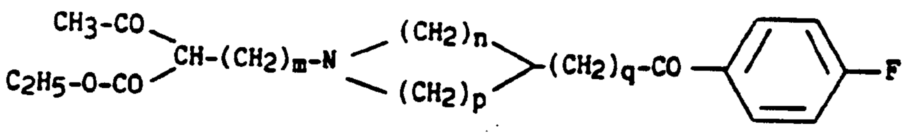

(VI)

wherein m, n, p and q are as defined for formula I according to claim 1,

to form the compounds of formula I according to claim 1 wherein R represents a radical of formula B and m, n, p and q are as defined for formula I,

which are then,

if desired, separated into their possible stereoisomers and/or converted into salts with a pharmaceutically acceptable organic or mineral acid to form the corresponding addition salts.

**2.** 8-cyano-3-{2-[4-(4-fluorobenzoyl)-piperidino]-ethyl}-4-hydroxy-2-methylimidazo[1,5-a]pyrimidine, a

compound corresponding to the general formula I according to claim 1, its optical isomers and its addition salts with a pharmaceutically acceptable mineral or organic acid, when prepared in accordance with the process of claim 1 or an equivalent chemical process.

**3.** 2,4-dioxo-3-{2-[3-(4-fluorophenacyl)-l-pyrrolidinyl]-ethyl}-1,2,3,4-tetrahydroquinazoline, a compound corresponding to the general formula I according to claim 1, its optical isomers and its addition salts with a pharmaceutically acceptable mineral or organic acid, when prepared in accordance with the process of claim 1 or an equivalent chemical process.

**4.** 8-carbamoyl-3-{2-[3-(4-fluorophenacyl)-1-pyrrolidinyl]-ethyl}-4-hydroxy-2-methylimidazo[1,5-a]pyrimid ine, a compound corresponding to the general formula I according to claim 1, its optical isomers and its addition salts with a pharmaceutically acceptable mineral or organic acid, when prepared in accordance with the process of claim 1 or an equivalent chemical process.

**5.** 1-{3-[4-(4-fluorobenzoyl)-piperidino]-propyl}-4-(2-fluorobenzyl)-piperazine-2,6-dione, a compound corresponding to the general formula I according to claim 1, its optical isomers and its addition salts with a pharmaceutically acceptable mineral or organic acid, when prepared in accordance with the process of claim 1 or an equivalent chemical process.

**6.** 1-{3-[4-(4-fluorobenzoyl)-piperidino]-propyl}-4-(2-pyrimidinyl)-piperazine, a compound corresponding to the general formula I according to claim 1, its optical isomers and its addition salts with a pharmaceutically acceptable mineral or organic acid, when prepared in accordance with the process of claim 1 or an equivalent chemical process.

**Claims for the following Contracting State : GR**

**1.** Process for the preparation of the compounds of the general formula I:

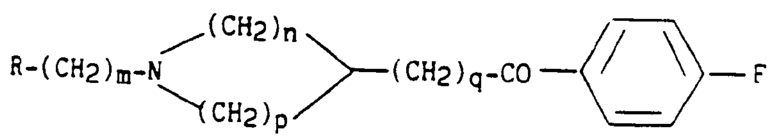

(I)

wherein:
- m represents an integer from 2 to 4,
- each of n and p, which may be identical or different, represents an integer from 1 to 3, but n and p do not simultaneously represent the number 3,
- q represents 0 or 1,

and

R represents:

. a grouping of formula (A):

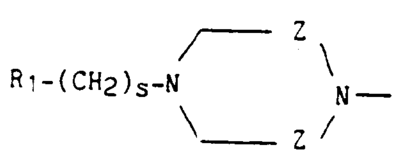

(A)

wherein s represents an integer from 0 to 4, Z represents a methylene radical or a carbonyl radical and $R_1$ represents either a phenyl radical (optionally substituted by one or more halogen atoms, by a linear or branched lower alkyl radical containing from 1 to 5 carbon atoms or a lower alkoxy radical containing from 1 to 5 carbon atoms) or a diphenylmethylene radical (optionally substituted by one or more halogen atoms, by a lower alkyl radical or a lower alkoxy radical), or an unsaturated ring having five or six ring members and comprising one or two nitrogen atoms,

. or a radical of formula (B):

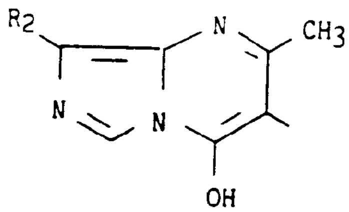

(B)

wherein $R_2$ represents a carbamoyl radical, a cyano radical, a carboxy radical or an alkoxycarbonyl radical containing from 2 to 7 carbon atoms,

. or a 2,4-dioxo-1,2,3,4-tetrahydroquinazolinyl radical with the proviso that, in that case, n and p do not simultaneously represent the number 2,

. or a 1-oxophthalazinyl radical, with the proviso that n and p do not simultaneously represent the number 2,

. or a 5-oxothiazolo[3,2-a]pyrimidinyl radical (optionally substituted by one or more halogen atoms, by a linear or branched alkyl radical containing from 1 to 5 carbon atoms or a lower alkoxy radical containing from 1 to 5 carbon atoms), with the proviso that n and p do not simultaneously represent the number 2,

. or a benzhydryloxy grouping (the phenyl radicals of which are optionally substituted by one or more halogen atoms, by a linear or branched alkyl radical containing from 1 to 5 carbon atoms or an alkoxy radical containing from 1 to 5 carbon atoms),

. or a grouping of the formula C:

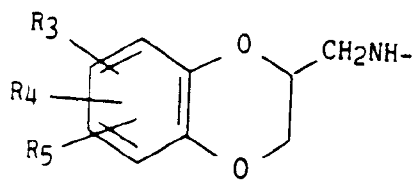

(C)

wherein each of $R_3$, $R_4$ and $R_5$, which may be identical or different, represents a halogen atom, an alkoxy radical having from 1 to 5 carbon atoms or a linear or branched alkyl radical having from 1 to 5 carbon atoms,

their possible stereoisomers, and their addition salts with a pharmaceutically acceptable mineral or organic acid,

characterised in that:

either:

a compound of the general formula II:

$$R\text{-}(CH_2)_m\text{-}X \qquad (II)$$

wherein R and m are as defined for formula I according to claim 1 and X represents a leaving group, such as a halogen atom, a mesyl radical or a tosyl radical, is condensed with an amine of the general formula III

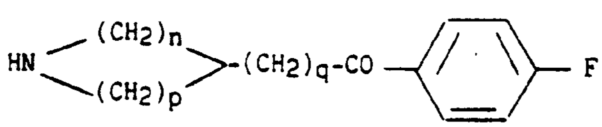

(III)

wherein n, p and q are as defined for formula I according to claim 1,

or

a compound of the general formula IV:

$$RH \qquad (IV)$$

wherein R is as defined for formula I according to claim 1,

is condensed with a compound of formula V:

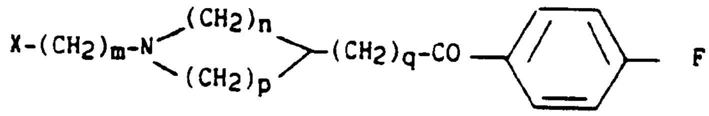

(V)

wherein m, n, p and q are as defined for formula I according to claim 1 and X represents a leaving group which is as defined for the general formula II, to form the compounds of formula I according to claim 1,

or:

a 4-aminoimidazole compound is cyclised with a compound of formula VI:

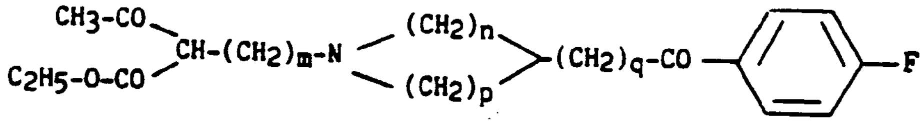

(VI)

wherein m, n, p and q are as defined for formula I according to claim 1, to form the compounds of formula I according to claim 1 wherein R represents a radical of formula B and m, n, p and q are as defined for formula I,

which are then,

if desired, separated into their possible stereoisomers and/or converted into salts with a pharmaceutically acceptable organic or mineral acid to form the corresponding addition salts.

**2.** 8-cyano-3-{2-[4-(4-fluorobenzoyl)-piperidino]-ethyl}-4-hydroxy-2-methylimidazo[1,5-a]pyrimidine, a compound corresponding to the general formula I according to claim 1, its optical isomers and its addition salts with a pharmaceutically acceptable mineral or organic acid, when prepared in accordance with the process of claim 1 or an equivalent chemical process.

**3.** 2,4-dioxo-3-{2-[3-(4-fluorophenacyl)-1-pyrrolidinyl]-ethyl}-1,2,3,4-tetrahydroquinazoline, a compound corresponding to the general formula I according to claim 1, its optical isomers and its addition salts with a pharmaceutically acceptable mineral or organic acid, when prepared in accordance with the process of claim 1 or an equivalent chemical process.

**4.** 8-carbamoyl-3-{2-[3-(4-fluorophenacyl)-1-pyrrolidinyl]-ethyl}-4-hydroxy-2-methylimidazo[1,5-a]pyrimidine, a compound corresponding to the general formula I according to claim 1, its optical isomers and its addition salts with a pharmaceutically acceptable mineral or organic acid, when prepared in accordance with the process of claim 1 or an equivalent chemical process.

**5.** 1-{3-[4-(4-fluorobenzoyl)-piperidino]-propyl}-4-(2-fluorobenzyl)-piperazine-2,6-dione, a compound corresponding to the general formula I according to claim 1, its optical isomers and its addition salts with a pharmaceutically acceptable mineral or organic acid, when prepared in accordance with the process of claim 1 or an equivalent chemical process.

**6.** 1-{3-[4-(4-fluorobenzoyl)-piperidino]-propyl}-4-(2-pyrimidinyl)-piperazine, a compound corresponding to the general formula I according to claim 1, its optical isomers and its addition salts with a pharmaceutically acceptable mineral or organic acid, when prepared in accordance with the process of claim 1 or an equivalent chemical process.